# EUROPÄISCHE PATENTSCHRIFT

(11) **EP 0 888 262 B1**
(45) Veröffentlichungstag und Bekanntmachung des Hinweises auf die Patenterteilung: **02.06.2004**
(21) Anmeldenummer: 97908184.1
(22) Anmeldetag: 06.03.1997
(51) Int. Cl.: C07B 61/00, C07C 33/12, C07C 33/14, C07C 33/36, C07C 69/608

(54) **CYCLOALKYLDERIVATE UND IHRE SYNTHESE AN FESTER PHASE**
CYCLOALKYL DERIVATIVES AND THE SOLID-PHASE SYNTHESIS OF SUCH DERIVATIVES
DERIVES DE CYCLOALKYLE ET LEUR SYNTHESE EN PHASE SOLIDE

(30) Priorität: 15.03.1996 DE 19610103
(43) Veröffentlichungstag der Anmeldung: 07.01.1999
(73) Patentinhaber: BASF AKTIENGESELLSCHAFT, 67056 Ludwigshafen (DE)
(72) Erfinder: STEINMETZ, Adrian, D-37081 Göttingen (DE); TIETZE, Lutz, F., D-37077 Göttingen (DE)
(86) Internationale Anmeldenummer: PCT/EP1997/001127
(87) Internationale Veröffentlichungsnummer: WO 1997/034852

(56) Entgegenhaltungen:
- US-A- 5 288 514
- ANGEWANDTE CHEMIE. INTERNATIONAL EDITION, Bd. 24, Nr. 12, Dezember 1985, WEINHEIM, DE, Seiten 1042-1043, XP002032269 L.F. TIETZE, ET AL.: "Non-induced, highly diastereoselective intramolecular ene reactions of 1,7-dienes to trans-1,2-disubstituted cyclohexanes"
- ANGEWANDTE CHEMIE. INTERNATIONAL EDITION, Bd. 27, Nr. 5, Mai 1988, WEINHEIM, DE, Seiten 703-705, XP002032270 L.F. TIETZE, ET AL.: "Stereoselective synthesis of trans-decalins via intramolecular ene reactions; synthesis of the enantiomerically pure cadinane-sesquiterpene veticadinol"
- ANGEWANDTE CHEMIE. INTERNATIONAL EDITION, Bd. 27, Nr. 9, September 1988, WEINHEIM, DE, Seiten 1186-1187, XP002032271 L.F. TIETZE, ET AL.: "Induced and non-induced diastereoselective intramolecular ene reaction of 1,6-dienes: the unusual formation of trans-1,2-disubstituted cyclopentanes"
- JOURNAL OF ORGANIC CHEMISTRY, Bd. 54, Nr. 13, 23.Juni 1989, WASHINGTON, DC, US, Seiten 3120-2129, XP002032272 L.F. TIETZE, ET AL.: "Asymmetric induction in intramolecular ene reactions of chiral 1,7-dienes: a diastereo- and enantioselective synthesis of substituted cyclohexanes"
- SYNTHESIS, Nr. 5, Mai 1988, STUTTGART, DE, Seiten 359-362, XP002032273 L.F. TIETZE, ET AL.: "New and efficient Lewis acid catalysts in intamolecular ene reactions"
- TETRAHEDRON LETTERS, Bd. 27, Nr. 16, 1986, OXFORD, GB, Seiten 1767-1770, XP002032274 L.F. TIETZE, ET AL.: "Asymmetric induction in intramolecular ene reactions of 1,7-dienes"
- TETRAHEDRON LETTERS, Bd. 31, Nr. 27, 1990, OXFORD, GB, Seiten 3857-3858, XP002032275 E.J. COREY, ET AL.: "A new enatiospecific route to the pseudoterosins"
- MOLECULAR CRYSTALS AND LIQUID CRYSTALS, Bd. 191, November 1990, READING, GB, Seiten 295-300, XP000206512 C. TSCHIERSKE, ET AL.: "Liquid crystalline properties of 2-(trans-4-n-alkylcyclohexyl)-propane-1,3- diols"
- TETRAHEDRON LETTERS, Bd. 35, Nr. 52, 24.Dezember 1993, OXFORD, GB, Seiten 8549-8552, XP002032276 G. GUANTI, ET AL.: "Chemoenzymatic approach to the AB ring system of Aklavinone"

## Beschreibung

Die Erfindung betrifft Cycloalkylderivate, ein Verfahren zu ihrer Herstellung und ihre Verwendung.

In der klassischen Wirkstoffsuchforschung wurde die biologische Wirkung neuer Verbindungen in einem Zufalls-Screening am ganzen Organismus beispielsweise der Pflanze oder dem Mikroorganismus getestet. Dabei war die biologische Testung gegenüber der Synthesechemie der limitierende Faktor. Durch die Bereitstellung molekularer Testsysteme durch die Molekular- und Zellbiologie hat sich die Situation drastisch verändert.

Für die moderne Wirkstoffsuchforschung wurden und werden zur Zeit eine Vielzahl von molekularen Testsystemen wie beispielsweise Rezeptorbindungsassays, Enzymassays und Zell-Zellinteraktionsassays entwickelt. Die Automatisierung und Miniaturisierung dieser Testsysteme ermöglicht einen hohen Probendurchsatz. Durch diese Entwicklung läßt sich in immer kürzerer Zeit eine immer größere Anzahl an Chemikalien auf ihre biologische Wirkung im Zufalls-Screening und damit auf eine mögliche Verwendung als Leitstruktur für einen Wirkstoff in der Medizin, Tiermedizin oder im Pflanzenschutz testen.

Ein modernes automatisiertes Testsystem ermöglicht in einem Massenscreening die Prüfung von 100.000 und mehr Chemikalien pro Jahr auf ihre biologische Wirkung.

Die klassische Synthesechemie wurde durch diese Entwicklung zum limitierenden Faktor in der Wirkstoffsuchforschung.

Soll die Leistungsfähigkeit dieser Testsysteme voll ausgeschöpft werden, muß die Effizienz der chemischen Wirkstoffleitstruktursynthese beträchtlich gesteigert werden.

Zu dieser erforderlichen Effizienzsteigerung kann die kombinatorische Chemie einen Beitrag leisten, insbesondere wenn sie sich automatisierter Festphasensynthesemethoden bedient (s. z.B. Übersichtsartikel J. Med. Chem. 1994, 37, 1233 und 1994, 37, 1385). Die kombinatorische Chemie ermöglicht die Synthese einer breiten Vielfalt unterschiedlicher chemischer Verbindungen, sogenannter Substanzbibliotheken. Die Synthese an der Festphase hat den Vorteil, daß Nebenprodukte und überschüssige Reaktanten leicht entfernt werden können, so daß keine aufwendige Reinigung der Produkte notwendig ist. Die fertigen Syntheseprodukte können direkt, d.h. trägergebunden, oder nach Abspaltung von der festen Phase dem Massenscreening zugeführt werden. Auch Zwischenprodukte können im Massenscreening geprüft werden.

Bisher beschriebene Anwendungen beschränken sich überwiegend auf das Peptid- und Nukleotidgebiet (Lebl et al., Int. J. Pept. Prot. Res. 41, 1993: 203 und WO 92/00091) oder ihre Derivate (WO 96/00391). Da Peptide und Nukleotide als Pharmaka wegen ihrer ungünstigen pharmakologischen Eigenschaften nur begrenzt einsetzbar sind, ist es wünschenswert, die von der Peptid- und Nukleotidchemie her bekannten und bewährten Festphasensynthesemethoden für die synthetische organische Chemie zu nutzen.

In US 5 288 514 wird über eine der ersten kombinatorischen Festphasensynthesen in der organischen Chemie außerhalb der Peptid- und Nukleotidchemie berichtet. US 5 288 514 beschreibt die sequentielle Synthese von 1,4-Benzodiazepinen an fester Phase.

WO 95/16712, WO 95/30642 und WO 96/00148 beschreiben weitere Festphasensynthesen potentieller Wirkstoffe in der kombinatorischen Chemie.

Um die Möglichkeiten der modernen Testsysteme im Massenscreening voll ausnutzen zu können, ist es jedoch notwendig, ständig neue Verbindungen mit einer möglichst hohen strukturellen Diversität in das Massenscreening einzuspeisen. Durch dieses Vorgehen läßt sich die Zeit zur Identifizierung und Optimierung einer neuen wirkstoffleitstruktur beträchtlich verkürzen.

Es ist deshalb erforderlich, ständig neue diverse, kombinatorische Festphasensynthesen zu entwickeln. Dabei ist es sinnvoll, sich an biologisch wirksamen Verbindungen zu orientieren.

Angesichts der Bedeutung von Cycloalkylderivaten speziell der Cycloalkylmalonsäureesterderivate als potentielle Wirkstoffe im Pharma- und Pflanzenschutzbereich ist es von großer Wichtigkeit, effiziente Methoden zu deren Herstellung an der festen Phase und insbesondere für die anschließende Testung im Massenscreening zur Verfügung zu stellen.

Aufgabe der vorliegenden Erfindung war es, ein schnelles und effizientes Herstellverfahren von Cycloalkylderivaten an der festen Phase bereitzustellen, das die Anforderungen der kombinatorischen Chemie erfüllt.

Es wurde nun ein Verfahren zur Herstellung von Cycloalkylderivaten der Formel I gefunden 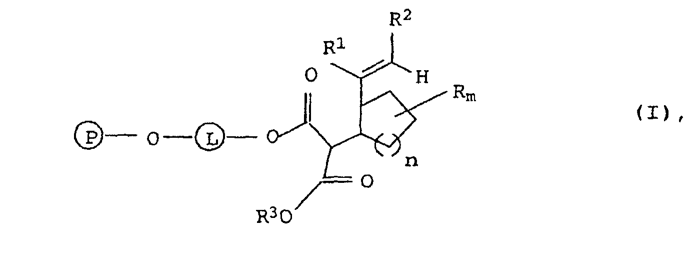 in der die Variablen und Substituenten folgende Bedeutung haben:
- (P): eine feste Phase, ausgewählt aus der Gruppe Keramik, Glas, Latex, quervernetzte Polystyrole, Polyacrylamide, Silicagele, Cellulosepartikel, Harze, Gold oder colloidale Metallpartikel,
- (L): einen Linker mit 2 bis 12 Kohlenstoffatomen oder der Struktur - CH₂-CH₂ (-O-CH₂-CH₂- )₁₋₁₀₀-,
- R¹: Wasserstoff oder einen niedermolekularen organischen Rest,
- R²: Wasserstoff oder gegebenenfalls substituiertes Alkyl, Alkenyl, Alkinyl oder Cycloalkyl oder
- R¹ und R²: zusammen einen gegebenenfalls substituierten 4 bis 8-gliedrigen Ring
- R³: ggf. subst. C₁-C₁₀-Alkyl, C₃-C₈-Cycloalkyl oder Aryl,
- R: einen niedermolekularen organischen Rest oder zwei benachbarte Reste R zusammen einen gegebenenfalls substituierten carbo- oder heterocyclischen Ring bilden
- n =: 0 bis 4
- m =: 0 bis n + 2,
das dadurch gekennzeichnet ist, daß man eine Verbindung der Formel II 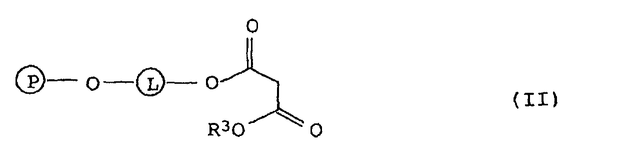 mit Aldehyden der Formel III 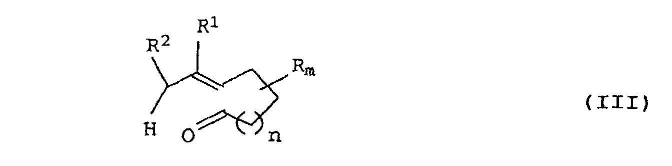 in Gegenwart einer Base zu Verbindungen der Formel IV 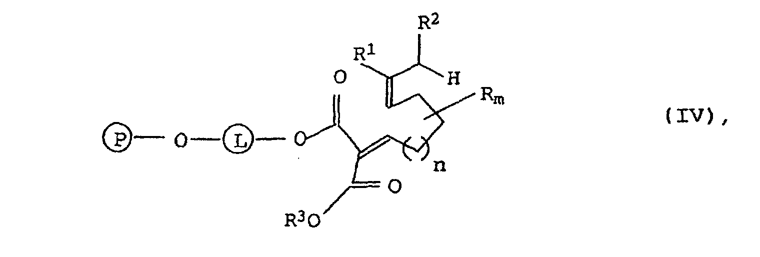 umsetzt, und das erhaltene Produkt IV in Gegenwart einer Lewis-Säure zyklisiert.

Die Erfindung betrifft außerdem neue Cycloalkylderivate und ihre Verwendung.

Als feste Phase (P) können in dem erfindungsgemäßen Verfahren Träger, wie sie aus der Festphasen-Peptidsynthese bekannt sind, verwendet werden. Nutzbare Träger können soweit sie mit der verwendeten Synthesechemie kompatibel sind aus einer Vielzahl von Materialien bestehen. Die Größe der Träger kann je nach Material in weitem Rahmen variiert werden. Bevorzugt werden Partikel im Bereich von 1 µm bis 1,5 cm als Träger verwendet, besonders bevorzugt bei polymeren Trägern Partikel im Bereich zwischen 1 µm und 100 µm.

Die Form der Träger ist beliebig, bevorzugt sind sphärische Partikel. Die Träger können in ihrer Größenverteilung homogen oder heterogen sein, bevorzugt sind homogene Partikelgrößen.

Geeignete feste Phasen (P) sind Keramik, Glas, Latex, quervernetzte Polystyrole, Polyacrylamide, Silicagele, Cellulosepartikel, Harze, Gold oder colloidale Metallpartikel.

Um eine Anknüpfung des Reaktanten bzw. eine Abspaltung des Syntheseproduktes nach der Synthese zu ermöglichen, muß der Träger geeignet funktionalisiert oder mit einem Linker versehen sein, der eine entsprechende funktionelle Gruppe besitzt. Bevorzugt geeignete Träger bzw. Träger-Linker-Konjugate sind beispielsweise Chlorbenzylharz (Merrifieldharz), Rink-Harz (Novabiochem), Sieber-Harz (Novabiochem), Wang-Harz (Bachem), Tentagel-Harze (Rapp-Polymere), Pega-Harz (Polymer Laboratories) oder Polyacrylamide. Besonders bevorzugt sind als Träger Chlorbenzylharze, Tentagel-Harze oder Polyacrylamide. Zur Anknüpfung des bevorzugten Linkers

HO-Ⓛ -OH

mit 2 bis 12 Kohlenstoffatomen an die feste Phase, ist diese gegebenenfalls in dem Fachmann bekannterweise zu modifizieren. Der Linker kann verzweigt oder unverzweigt, chiral oder achiral sein.

Als Diole seien beispielsweise Ethylenglycol, 1,3- Propandiol, 1,2-Propandiol, 1,2-Butandiol, 1,3-Butandiol, 1,4-Butandiol, 2,3-Butandiol, 1,2-Pentandiol, 1,3-Pentandiol, 1,4-Pentandiol, 1,5-Pentandiol, 2,4-Pentandiol, 2-Methyl-1,4-Butandiol, 1,2-Hexandiol, 1,3-Hexandiol, 1,4-Hexandiol, 1,5-Hexandiol, 1,6-Hexandiol, 2,3-Hexandiol, 2,4-Hexandiol, 2,5-Hexandiol, 2-Methyl-1,5-Pentandiol oder 3-Methyl-1,5-Pentandiol genannt.

Zur Bestimmung der Konzentration der Hydroxygruppen des Linkerverknüpften Harzes wurde dieses mit 3,5-Dinitrobenzoylchlorid in Pyridin umgesetzt, die Stickstoffbestimmung des erhaltenen Esters ist ein Maß für die Hydroxygruppenkonzentration. Diese liegt im Bereich von 0,5 bis 0,85 mmol Hydroxygruppen pro Gramm Harz.

Polyacrylamide [(P)-NH₂] können beispielsweise mit 4-Chlormethylbenzoesäure so derivatisiert werden, daß der zweifachdeprotonierte Linker angeknüpft werden kann (Schema I).

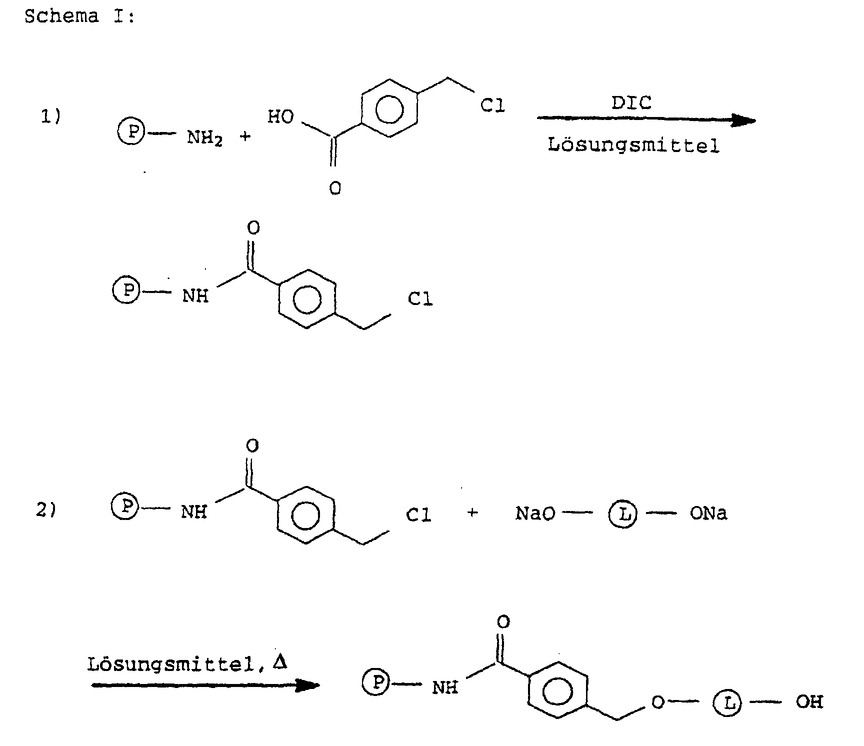

Die Amidbindunq (1) zwischen dem Träger und der 4-Chlormethylbenzoesäure kann im Lösungsmittel mit Hilfe von beispielsweise Diisopropylcarbodiimid (=DIC) geknüpft werden. Weitere zur Knüpfung der Amidbindung geeigneten Kupplungsreagenzien sind beispielsweise TBTU, HBTU, BOP oder PYBOP (Lit.: Int. J. Peptide Prot. Rev. 35, 1990: 161-214).

Als Lösungsmittel für die Bildung der funktionalisierten festen Phase eignen sich aprotische, unpolare oder polare Lösungsmittel, beispielsweise DMF, CH₂Cl₂, DMSO oder THF. Es können einzelne Lösungsmittel oder Gemische verwendet werden.

Die Kupplung des bevorzugten Linkers an Merifieldharz kann direkt in der doppelt-deprotonierten Form des Linkers (Schema I,2) in Gegenwart der oben beschriebenen Lösungsmittel erfolgen.

Die Reaktion (2) wird zwischen 30 und 150°C, bevorzugt zwischen 60 und 100°C, durchgeführt.

Die Abspaltung des Syntheseprodukts I von der festen Phase kann durch Reduktion, Umesterung, Amidierung oder Basenkatalyse erfolgen.

R¹ bezeichnet in den Verbindungen der Formeln I, III, IV und VI Wasserstoff oder einen niedermolekularen organischen Rest, der 1 bis 26 Kohlenstoffatome neben ggf. mindestens einem Sauerstoff- und/oder mindestens einem Heteroatom enthalten kann.

Als niedermolekulare organische Reste seien für R¹ gegebenenfalls substituierte Alkyl-, Alkenyl-, Alkinyl-, Cycloalkyl-, Cycloalkenyl-, Cycloalkinyl-, Aryl-, Hetaryl-, Alkylaryl-, Alkylhetaryl-, Alkylcarbonyl-, Arylcarbonyl-, Alkylsulfonyl- oder Arylsulfonylreste genannt.

Als Alkylreste seien verzweigte oder unverzweigte C₁-C₈-Alkylketten wie beispielsweise Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl oder n-Octyl genannt.

Als Substituenten kommen ein oder mehrere Substituenten wie Halogen, wie Fluor, Chlor oder Brom, Cyano, Nitro, Amino, Hydroxy, Thio, Alkyl, Aryl, Alkoxy, Carboxy, Benzyloxy, Phenyl oder Benzyl in Frage.

Als Alkenylreste seien verzweigte oder unverzweigte C₂-C₈-Alkenylketten wie beispielsweise Ethenyl, Propenyl, 1-Butenyl, 2-Butenyl, 3-Butenyl, 2-Methylpropenyl, 1-Pentenyl, 2-Pentenyl, 3-Pentenyl, 4-Pentenyl, 1-Methyl.-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl,1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3 -Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-Pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl, 1-Ethyl-2-methyl-2-propenyl, 1-Heptenyl, 2-Heptenyl, 3-Heptenyl, 4-Heptenyl, 5-Heptenyl, 6-Heptenyl, 1-Octenyl, 2-Octenyl, 3-Octenyl, 4-Octenyl, 5-Octenyl, 6-Octenyl, 7-Octenyl genannt.

Als Substituenten kommen ein oder mehrere Substituenten wie Halogen wie Fluor, Chlor oder Brom, Cyano, Nitro, Amino, Hydroxy,

Thio, Alkyl, Aryl, Alkoxy, Carboxy, Benzyloxy, Phenyl oder Benzyl in Frage.

Unter Alkinyl sind C₂-C₆-Alkinyl-Reste wie Ethinyl, Prop-1-in-1-yl, Prop-2-in-1-yl, n-But-1-in-1-yl, n-But-1-in-3-yl, n-But-1-in-4-yl, n-But-2-in-1-yl, n-Pent-1-in-1-yl, n-Pent-1-in-3-yl, n-Pent-1-in-4-yl, n-Pent-1-in-5-yl, n-Pent-2-in-1-yl, n-Pent-2-in-4-yl, n-Pent-2-in-5-yl, 3-Methyl-but-1-in-3-yl, 3-Methyl-but-1-in-4-yl, n-Hex-1-in-1-yl, n-Hex-1-in-3-yl, n-Hex-1-in-4-yl, n-Hex-1-in-5-yl, n-Hex-1-in-6-yl, n-Hex-2-in-1-yl, n-Hex-2-in-4-yl, n-Hex-2-in-5-yl, n-Hex-2-in-6-yl, n-Hex-3-in-1-yl, n-Hex-3-in-2-yl, 3-Methyl-pent-1-in-1-yl, 3-Methyl-pent-1-in-3-yl, 3-Methyl-pent-1-in-4-yl, 3-Methyl-pent-1-in-5-yl, 4-Methyl-pent-1-in-1-yl, 4-Methyl-pent-2-in-4-yl oder 4-Methyl-pent-2-in-5-yl zu verstehen.

Der Alkinylrest kann gegebenenfalls mit einem oder mehreren Substituenten wie Halogen, wie Fluor, Chlor oder Brom, Cyano, Nitro, Amino, Hydroxy, Thio, Alkyl, Aryl, Alkoxy, Carboxy, Benzyloxy, Phenyl oder Benzyl substituiert sein.

Als Cycloalkylreste seien C₃-C₈-Cycloalkylketten wie Cyclopropyl, Cyclobutyl, Cycloheptyl, Cyclohexyl, Cycloheptyl oder Cyclooctyl genannt.

Als Substituenten kommen ein oder mehrere Reste wie Halogen, wie Fluor, Chlor oder Brom, Cyano, Nitro, Amino, Hydroxy, Thio, Alkyl, Aryl, Alkoxy, Carboxy, Benzyloxy, Phenyl oder Benzyl in Frage.

Unter Arylresten sind einfache oder kondensierte aromatische Ringsysteme zu verstehen, die gegebenenfalls mit einem oder mehreren Resten wie Halogen wie Fluor, Chlor oder Brom, Cyano, Nitro, Amino, Hydroxy, Thio, Alkyl, Alkoxy oder anderen Resten substituiert sein können.

Bevorzugte Arylreste sind Phenyl oder Naphthyl.

Unter Hetarylresten sind einfache oder kondensierte aromatische Ringsysteme mit einem oder mehreren heteroaromatischen 3- bis 7-gliedrigen Ringen zu verstehen, die gegebenenfalls mit einem oder mehreren Resten wie Halogen wie Fluor, Chlor oder Brom, Cyano, Nitro, Amino, Hydroxy, Thio, Alkyl, Alkoxy oder anderen Resten substituiert sein können.

Als Heteroatome können ein oder mehrere Stickstoff-, Schwefel- und/oder Sauerstoffatome im Ring oder Ringsystem enthalten sein.

Als Alkylarylreste seien verzweigtkettige oder unverzweigtkettige C₁-C₆-Alkyl-phenyl- oder C₁-C₆-Alkyl-naphthylreste wie Methylphenyl, Ethylphenyl, Propylphenyl, 1-Methylethylphenyl, Butylphenyl, 1-Methylpropylphenyl, 2-Methylpropylphenyl, 1,1-Dimethylethylphenyl, Pentylphenyl, 1-Methylbutylphenyl, 2-Methylbutylphenyl, 3-Methylbutylphenyl, 2,2-Dimethylpropylphenyl, 1-Ethylpropylphenyl, n-Hexylphenyl, 1,1-Dimethylpropylphenyl, 1,2-Dimethylpropylphenyl, 1-Methylpentylphenyl, 2-Methylpentylphenyl, 3-Methylpentylphenyl, 4-Methylpentylphenyl, 1,1-Dimethylbutylphenyl, 1,2-Dimethylbutylphenyl, 1,3-Dimethylbutylphenyl, 1,2-Dimethylbutylphenyl, 1,3-Dimethylbutylphenyl, 2,2-Dimethylbutylphenyl, 2,3-Dimethylbutylphenyl, 3,3-Dimethylbutylphenyl, 1-Ethylbutylphenyl, 2-Ethylbutylphenyl, 1, 1,2-Trimethylpropylphenyl, 1,2,2-Trimethylpropylphenyl, 1-Ethyl-2-methylpropylphenyl, 1-Ethyl-2-methylpropylphenyl, Methylnaphthyl, Ethylnaphthyl, Propynaphthyl, 1-Methylethylnaphthyl, Butylnaphthyl, 1-Methylpropylnaphthyl, 2-Methylpropylnaphthyl, 1,1-Dimethylethylnaphthyl, Pentylnaphthyl, 1-Methylbutylnaphthyl, 2-Methylbutylnaphthyl, 3-Methylbutylnaphthyl, 2,2-Dimethylpropylnaphthyl, 1-Ethylpropylnaphthyl, n-Hexylnaphthyl, 1,1-Dimethylpropylnaphthyl, 1,2-Dimethylpropylnaphthyl, 1-Methylpentylnaphthyl, 2-Methylpentylnaphthyl, 3-Methylpentylnaphthyl, 4-Methylpentylnaphthyl, 1,1-Dimethylbutylnaphthyl, 1,2-Dimethylbutylnaphthyl, 1,3-Dimethylbutylnaphthyl, 1,2-Dimethylbutylnaphthyl, 1,3-Dimethylbutylnaphthyl, 2,2-Dimethylbutylnaphthyl, 2,3-Dimethylbutylnaphthyl, 3,3-Dimethylbutylnaphthyl, 1-Ethylbutylnaphthyl, 2-Ethylbutylnaphthyl, 1,1,2-Trimethylpropylnaphthyl, 2,2,2-Trimethylpropylnaphthyl, 1-Ethyl-1-methylpropylnaphthyl, 1-Ethyl-2-methylpropylnaphthyl, genannt.

Die Alkylarylreste können gegebenenfalls mit einem oder mehreren Resten wie Halogen wie Fluor, Chlor oder Borm, Cyano, Nitro, Amino, Hydroxy, Thio, Alkyl, Alkoxy oder anderen Resten substituiert sein.

Unter Alkylhetaryl sind verzweigtkettige oder unverzweigtkettige C₁-C₈-Alkylhetarylreste, die ein oder mehrere Stickstoff-, Schwefel- und/oder Sauerstoffatome im Ring oder Ringsystem enthalten, zu verstehen.

Die Alkylheterylreste können gegebenenfalls mit einem oder mehreren Resten wie Halogen wie Fluor, Chlor oder Brom, Cyano, Nitro, Amino, Hydroxy, Thio, Alkyl, Alkoxy oder anderen Resten substituiert sein.

Als Alkylcarbonylreste seien verzweigte oder unverzweigte C₁-C₄-Alkylcarbonylketten wie Acetyl, Ethylcarbonyl, n-Propylcarbonyl, 1-Methylethylcarbonyl, n-Butylcarbonyl, 1-Methylpropylcarbonyl, 2-Methylpropylcarbonyl oder 1,1-Dimethylethylcarbonyl genannt.

Als Arylcarbonylrest kommen Benzoyl oder Naphthoyl in Frage.

Alkylsulfonyl steht beispielsweise für Methylsulfonyl, Ethylsulfonyl, n-Propylsulfonyl, 1-Methylethylsulfonyl, n-Butylsulfonyl, 1-Methylpropylsulfonyl, 2-Methylpropylsulfonyl oder 1,1-Dimethylethylsulfonyl.

Als Arylsulfonylreste seien Phenylsulfonyl oder Naphthylsulfonyl genannt.

Alle Alkylcarbonyl-, Arylcarbonyl-, Alkylsulfonyl- und Arylsulfonylreste können gegebenenfalls mit einem oder mehreren Resten wie Halogen wie Fluor, Chlor oder Brom, Cyano, Nitro, Amino, Hydroxy, Thio, Alkyl, Alkoxy oder anderen Resten substituiert sein.

Für R² in den Verbindungen der Formeln I, III, IV oder VI seien als Reste Wasserstoff oder gegebenenfalls subst. Alkyl, Alkenyl, Alkinyl oder Cycloalkyl genannt, wobei
Alkyl verzweigte oder unverzweigte C₁-C₈-Alkylketten wie beispielsweise Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl oder n-Octyl;
Alkenyl verzweigte oder unverzweigte C₂-C₈-Alkenylketten wie beispielsweise Ethenyl, Propenyl, 1-Butenyl, 2-Butenyl, 3-Butenyl, 2-Methylpropenyl, 1-Pentenyl, 2-Pentenyl, 3-Pentenyl, 4-Pentenyl, 2-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-2-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 2,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1 -Ethyl-1-methyl-2-propenyl, 2-Ethyl-2-methyl-1-propenyl, 1-Ethyl-2-methyl-2-propenyl, 1-Heptenyl, 2-Heptenyl, 3-Heptenyl, 4-Heptenyl, 5-Heptenyl, 6-Heptenyl, 1-Octenyl, 2-Octenyl, 3-Octenyl, 4-Octenyl, 5-Octenyl, 6-Octenyl, 7-Octenyl.
- Alkinyl verzweigte oder unverzweigte C₂-C₆-Alkinylketten wie beispielswiese Ethinyl, Prop-1-in-1-yl, Prop-2-in-1-yl, n-But-1-in-1-yl, n-But-1-in-3-yl, n-But-1-in-4-yl, n-But-2-in-1-yl, n-Pent-1-in-1-yl, n-Pent-1-in-3-yl, n-Pent-1-in-4-yl, n-Pent-1-in-5-yl, n-Pent-2-in-1-yl, n-Pent-2-in-4-yl, n-Pent-2-in-5-yl, 3-Methyl-but-1-in-3-yl, 3-Methyl-but-1-in-4-yl, n-Hex-1-in-1-yl, n-Hex-1-in-3-yl, n-Hex-1-in-4-yl, n-Hex-1-in-5-yl, n-Hex-1-in-6-yl, n-Hex-2-in-1-yl, n-Hex-2-in-4-yl, n-Hex-2-in-5-yl, n-Hex-2-in-6-yl, n-Hex-3-in-1-yl, n-Hex-3-in-2-yl, 3-Methyl-pent-1-in-1-yl, 3-Methyl-pent-1-in-3-yl, 3-Methyl-pent-1-in-4-yl, 3-Methyl-pent-1-in-5-yl, 4-Methyl-pent-1-in-1-yl, 4-Methyl-pent-2-in-4-yl oder 4-Methyl-pent-2-in-5-yl und
- Cycloalkyl C₃-C₈-Cycloalkylketten wie beispielsweise Cyclopropyl, Cyclobutyl, Cycloheptyl, Cyclohexyl, Cycloheptyl oder Cyclooctyl bedeuten.

R¹ und R² können zusammen einen gegebenenfalls substituierten 4- bis 8-gliedrigen Ring bilden.

Alle genannten Reste R² können gegebenenfalls mit mindestens einem weiteren Rest aus der Gruppe Halogen wie Fluor, Chlor oder Brom, Cyano, Nitro, Amino, Hydroxy, Thio oder weiteren Resten substituiert sein.

Für R³ in den Verbindungen der Formeln I, II, IV, VI und VII seien als Reste gegebenenfalls subst. C₁-C₁₀-Alkyl, C₃-C₈-Cycloalkyl oder Aryl genannt, wobei
- Alykl verzweite oder unverzweigte C₁-C₁₀-Alkylketten, wie beispielsweise Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 2-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, n-Nonyl oder n-Decyl, wobei der Alkyrest mit einem ggf. subst. aromatischen oder heteroaromatischen Ring substituiert sein kann;
- Cycloalkyl beispielsweise Cyclopropan. Cyclobutan, Cyclopentan, Cyclohexan, Cycloheptan oder Cyclooctan
- Aryl Phenyl oder Naphthyl bedeutet.

Alle genannten Reste können gegebenenfalls mit mindestens einem weiteren Rest aus der Gruppe Halogen wie Fluor, Chlor oder Brom, Cyano, Nitro, Amino, Hydroxy, Thio oder weiteren Resten substituiert sein.

R bezeichnet in den Verbindungen der Formel I, III, IV und VI einen niedermolekularen organischen Rest, der 1 bis 26 Kohlenstoffatome neben ggf. mindestens einem Sauerstoff- und/oder mindestens einem Heteroatom enthalten kann.

Als niedermolekulare organische Reste seien für R gegebenenfalls substituierte Alkyl, Alkenyl, Alkinyl, Cycloalkyl, Aryl, Hetaryl, Alkylaryl, Alkylhetaryl, Alkylcarbonyl, Arylcarbonyl, Alkylsulfonyl oder Arylsulfonyl genannt.

Als Alkylreste seien verzweigte oder unverzweigte C₁-C₈-Alkylketten wie beispielsweise Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 2-Ethyl-2-methylpropyl, n-Heptyl oder n-Octyl genannt.

Als Substituenten kommen ein oder mehrere Substituenten wie Halogen, wie Fluor, Chlor oder Brom, Cyano, Nitro, Amino, Hydroxy, Thio, Alkyl, Aryl, Alkoxy, Carboxy, Benzyloxy, Phenyl oder Benzyl in Frage.

Als Alkenylreste seien verzweigte oder unverzweigte C₂-C₈-Alkenylketten wie beispielsweise Ethenyl, Propenyl, 1-Butenyl, 2-Butenyl, 3-Butenyl, 2-Methylpropenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-2-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl, 2-Ethyl-2-methyl-2-propenyl, 1-Heptenyl, 2-Heptenyl, 3-Heptenyl, 4-Heptenyl, 5-Heptenyl, 6-Heptenyl, 1-Octenyl, 2-Octenyl, 3-Octenyl, 4-Octenyl, 5-Octenyl, 6-Octenyl, 7-Octenyl genannt.

Als Substituenten kommen ein oder mehrere Substituenten wie Halogen wie Fluor, Chlor oder Brom, Cyano, Nitro, Amino, Hydroxy, Thio, Alkyl, Aryl, Alkoxy, Carboxy, Benzyloxy, Phenyl oder Benzyl in Frage.

Unter Alkinyl sind C₂-C₆-Alkinyl-Reste wie Ethinyl, Prop-1-in-1-yl, Prop-2-in-1-yl, n-But-1-in-1-yl, n-But-1-in-3-yl, n-But-1-in-4-yl, n-But-2-in-1-yl, n-Pent-1-in-1-yl, n-Pent-1-in-3-yl, n-Pent-1-in-4-yl, n-Pent-1-in-5-yl, n-Pent-2-in-1-yl, n-Pent-2-in-4-yl, n-Pent-2-in-5-yl, 3-Methyl-but-1-in-3-yl, 3-Methyl-but-1-in-4-yl, n-Hex-1-in-1-yl, n-Hex-1-in-3-yl, n-Hex-1-in-4-yl, n-Hex-1-in-5-yl, n-Hex-1-in-6-yl, n-Hex-2-in-1-yl, n-Hex-2-in-4-yl, n-Hex-2-in-5-yl, n-Hex-2-in-6-yl, n-Hex-3-in-1-yl, n-Hex-3-in-2-yl, 3-Methyl-pent-1-in-1-yl, 3-Methyl-pent-1-in-3-yl, 3-Methyl-pent-1-in-4-yl, 3-Methyl-pent-1-in-5-yl, 4-Methyl-pent-1-in-1-yl, 4-Methyl-pent-2-in-4-yl oder 4-Methyl-pent-2-in-5-yl zu verstehen.

Der Alkinylrest kann gegebenenfalls mit einem oder mehreren Substituenten wie Halogen, wie Fluor, Chlor oder Brom, Cyano, Nitro, Amino, Hydroxy, Thio, Alkyl, Aryl, Alkoxy, Carboxy, Benzyloxy, Phenyl oder Benzyl substituiert sein.

Als Cycloalkylreste seien C₃-C₈-Cycloalkylketten wie Cyclopropyl, Cyclobutyl, Cycloheptyl, Cyclohexyl, Cycloheptyl oder Cyclooctyl genannt.

Als Substituenten kommen ein oder mehrere Reste wie Halogen, wie Fluor, Chlor oder Brom, Cyano, Nitro, Amino, Hydroxy, Thio, Alkyl, Aryl, Alkoxy, Carboxy, Benzyloxy, Phenyl oder Benzyl in Frage.

Unter Arylresten sind einfache oder kondensierte aromatische Ringsysteme zu verstehen, die gegebenenfalls mit einem oder mehreren Resten wie Halogen wie Fluor, Chlor oder Brom, Cyano, nitro, Amino, Hydroxy, Thio, Alkyl, Alkoxy oder anderen Resten substituiert sein können.

Bevorzugte Arylreste sind Phenyl oder Naphthyl.

Unter Hetarylresten sind einfache oder kondensierte aromatische Ringsysteme mit einem oder mehreren heteroaromatischen 3- bis 7-gliedringen Ringen zu verstehen, die gegebenenfalls mit einem oder mehreren Resten wie Halogen wie Fluor, Chlor oder Brom, Cyano, Nitro, Amino, Hydroxy, Thio, Alkyl, Alkoxy oder anderen Resten substituiert sein können.

Als Heteroatome können ein oder mehrere Stickstoff-, Schwefel- und/oder Sauerstoffatome im Ring oder Ringsystem enthalten sein.

Als Alkylarylreste seien verzweigtkettige oder unverzweigtkettige C₁-C₆-Alkyl-phenyl- oder C₁-C₆-Alkyl-naphthylreste wie Methylphenyl, Ethylphenyl, Propylphenyl, 1-Methylethylphenyl, Butylphenyl, 1-Methylpropylphenyl, 2-Methylpropylphenyl, 1,1-Dimethylethylphenyl, Pentylphenyl, 1-Methylbutylphenyl, 2-Methylbutyl-. phenyl, 3-Methylbutylphenyl, 2,2-Dimethylpropylphenyl, 1-Ethylpropylphenyl, n-Hexylphenyl, 1,1-Dimethylpropylphenyl, 1,2-Dimethylpropylphenyl, 1-Methylpentylphenyl, 2-Methylpentylphenyl, 3-Methylpentylphenyl, 4-Methylpentylphenyl, 1,1-Dimethylbutylphenyl, 1,2-Dimethylbutylphenyl, 1,3-Dimethylbutylphenyl, 1,2-Dimethylbutylphenyl, 1,3-Dimethylbutylphenyl, 2,2-Dimethylbutylphenyl, 2,3-Dimethylbutylphenyl, 3,3-Dimethylbutylphenyl, 2-Ethylbutylphenyl, 2-Ethylbutylphenyl, 1,1,2-Trimethylpropylphenyl, 1,2,2-Trimethylpropylphenyl, 1-Ethyl-1-methylpropylphenyl, 1-Ethyl-2-methylpropylphenyl, Methylnaphthyl, Ethylnaphthyl, Propynaphthyl, 1-Methylethylnaphthyl, Butylnaphthyl, 1-Methylpropylnaphthyl, 2-Methylpropylnaphthyl, 1,1-Dimethylethylnaphthyl, Pentylnaphthyl, 1-Methylbutylnaphthyl, 2-Methylbutylnaphthyl, 3-Methylbutylnaphthyl, 2,2-Dimethylpropylnaphthyl, 1-Ethylpropylnaphthyl, n-Hexylnaphthyl, 1,1-Dimethylpropylnaphthyl, 1,2-Dimethylpropylnaphthyl, 1-Methylpentylnaphthyl, 2 -Methylpentylnaphthyl, 3-Methylpentylnaphthyl, 4-Methylpentylnaphthyl, 1,1-Dimethylbutylnaphthyl, 1,2-Dimethylbutylnaphthyl, 1,3-Dimethylbutylnaphthyl, 1,2-Dimethylbutylnaphthyl, 1,3-Dimethylbutylnaphthyl, 2,2-Dimethylbutylnaphthyl, 2,3-Dimethylbutylnaphthyl, 3,3-Dimethylbutylnaphthyl, 1-Ethylbutylnaphthyl, 2-Ethylbutylnaphthyl, 1,1,2-Trimethylpropylnaphthyl, 1,2,2-Trimethylpropylnaphthyl, 1-Ethyl-1-methylpropylnaphthyl, 1-Ethyl-2-methylprogylnaphthyl, genannt.

Die Alkylarylreste können gegebenenfalls mit einem oder mehreren Resten wie Halogen wie Fluor, Chlor oder Borm, Cyano, Nitro, Amino, Hydroxy, Thio, Alkyl, Alkoxy oder anderen Resten substi-tuiert sein.

Unter Alkylhetaryl sind verzweigtkettige oder unverzweigtkettige C₁-C₈-Alkylhetarylreste, die ein oder mehrere Stickstoff-, Schwefel- und/oder Sauerstoffatome im Ring oder Ringsystem enthalten, zu verstehen.

Die Alkylhetarylreste können gegebenenfalls mit einem oder mehreren Resten wie Halogen wie Fluor, Chlor oder Brom, Cyano, Nitro, Amino, Hydroxy, Thio, Alkyl, Alkoxy oder anderen Resten substituiert sein.

Als Alkylcarbonylreste seien verzweigte oder unverzweigte C₁-C₄-Alkylcarbonylketten wie Acetyl, Ethylcarbonyl, n-Propylcarbonyl, 1-Methylethylcarbonyl, n-Butylcarbonyl, 1-Methylpropylcarbonyl, 2-Methylpropylcarbonyl oder 1,1-Dimethylethylcarbonyl genannt.

Als Arylcarbonylrest kommen Benzoyl oder Naphthoyl in Frage.

Alkylsulfonyl steht beispielsweise für Methylsulfonyl, Ethylsulfonyl, n-Propylsulfonyl, 1-Methylethylsulfonyl, n-Butylsulfonyl, 1-Methylpropylsulfonyl, 2-Methylpropylsulfonyl oder 1,1-Dimethylethylsulfonyl.

Als Arylsulfonylreste seien Phenylsulfonyl oder Naphthylsulfonyl genannt.

Alle Alkylcarbonyl-, Arylcarbonyl-, Alkylsulfonyl- und Arylsulfonylreste können gegebenenfalls mit einem oder mehreren Resten wie Halogen wie Fluor, Chlor oder Brom, Cyano, Nitro, Amino, Hydroxy, Thio, Alkyl, Alkoxy oder anderen Resten substituiert sein.

Zwei benachbarte Reste R können zusammen einen gegebenenfalls substituierten carbo- oder heterocyclischen Ring bilden. Die Ringe können gesättigt, mit mindestens einer Doppelbindung ungesättigt oder aromatisch sein. Die Ringe können 3- bis 8-gliedrig sein.

Als Heteroatom können ein oder mehrere Stickstoff-, Schwefel- und/oder Sauerstoffatome im Ring enthalten sein.

Als Substituenten der Ringe seien beispielsweise Halogen wie Fluor, Chlor oder Brom, Cyano, Nitro, Amino, Hydroxy oder Thio genannt.

Die Variable n steht für 0 bis 4, bevorzugt für 1 oder 2.

Die Variable m des Restes R steht für 0 bis n + 2.

Das erfindungsgemäße Verfahren zur Herstellung der Cycloalkylderivate kann in drei getrennten Schritten entsprechend Schema II durchgeführt werden; oder aber alle drei Schritte können in einer aufeinanderfolgenden Sequenz zusammen durchgeführt werden.

Das erfindungsgemäße Verfahren kann in einer Reihe paralleler automatisierter Syntheseansätze durchgeführt werden. Auch Reaktantengemische können in einem Syntheseansatz oder parallelen Syntheseansätzen eingesetzt werden.

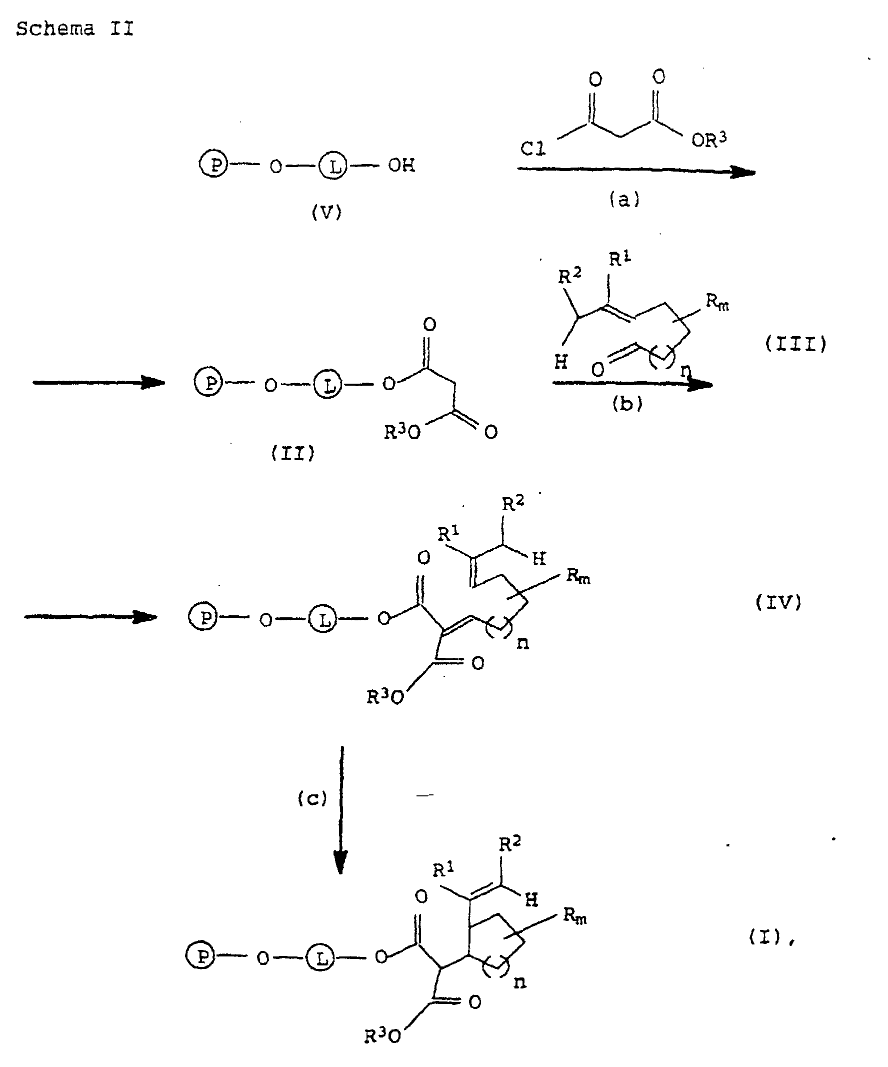

Die Synthese kann auch auf der Stufe des Produktes IV abgebrochen werden und das Produkt direkt oder nach Abspaltung isoliert und im Massenscreening getestet werden.

Reaktion (a) wird in Gegenwart einer Base, bevorzugt tert.-Aminbasen wie (iPr)₂NEt, Pyridin, NEt₃ oder DBU, durchgeführt. Als Lösungsmittel sind beliebige aprotische Lösungsmittel, beispielsweise DMF, THF, CH₂Cl₂ oder deren Gemische, zu nennen. Die Reaktion wird in einem Temperaturbereich von -20 bis +40°C. bevorzugt von -10 bis +10°, durchgeführt. Reaktion (b) wird in einem Temperaturbereich von +10 bis +130°C, bevorzugt +20 bis +70°C, in Gegenwart von Salzen aus Aminen und Carbonsäuren, wie beispielsweise EDDA (Ethylendiamin Diacetat) oder Piperidiniumacetat, durchgeführt. Gegebenenfalls ist der Zusatz wasserentziehender Mittel wie Na₂SO₄ oder Orthoester von Vorteil.

Die anschließende Zyklisierung (Reaktion (c)) erfolgt in Gegenwart einer Lewis-Säure in einem Temperaturbereich von -10 bis +130°C, bevorzugt von 0 bis +40°C. Die Zyklisierung kann aber auch rein thermisch ohne Anwesenheit eines Katalysators erfolgen. Bevorzugte Lewis-Säuren sind AlCl₃, AlBr₃, ZnBr₂, ZnCl₂, BF₃, BF₂x0Et₂, SnCl₄, Et₂AlCl oder TiCl₄. Als Lösungsmittel für die Reaktionen (a) und (b) können beliebige aprotische gegen Lewis-Säuren stabile Lösungsmittel verwendet werden.

Ist in der Verbindung III keine olefinische Doppelbindung enthalten und kann deshalb die anschließende Zyklisierung nicht durchgeführt werden, so kann ein externes Olefin mit dem Reaktionsprodukt aus Reaktion (b) umgesetzt werden.

Verbindung II kann als CH-acide Verbindung prinzipiell für alle bekannten C-C-Verknüpfungsreaktionen CH-acider Verbindungen (Organikum, Barth Verlagsgesellschaft mbH, 1993, 459-503) wie die Michaeladdition, die Aldolkondensation, die Robinson-Anellierung, Palladium-katalysierte allylische Substitutionen oder die Knoevenagel-Reaktion verwendet werden. Bevorzugte Reaktion ist die Knoevenagel-Reaktion.

Produkt I kann direkt oder nach Abspaltung vom Träger dem Massenscreening zugeführt werden.

Die Abspaltung des Syntheseprodukts I von der festen Phase kann durch Reduktion, Umesterung, Amidierung oder Basenkatalyse erfolgen (Schema III). Reaktion (d) wird geeigneterweise in einem Temperaturbereich von -20 bis +40°C, bevorzugt -10 bis +20°C, in Gegenwart eines Lösungsmittels und eines geeigneten Reduktionsmittels, beispielsweise DIBAH, LiAlH₄ oder LiBH₄ durchgeführt. Als Produkt entstehen die symmetrischen Diole. Als Lösungsmittel sind beliebige aprotische oder protische Lösungsmittel wie THF, Ether, MeOH oder Toluol geeignet.

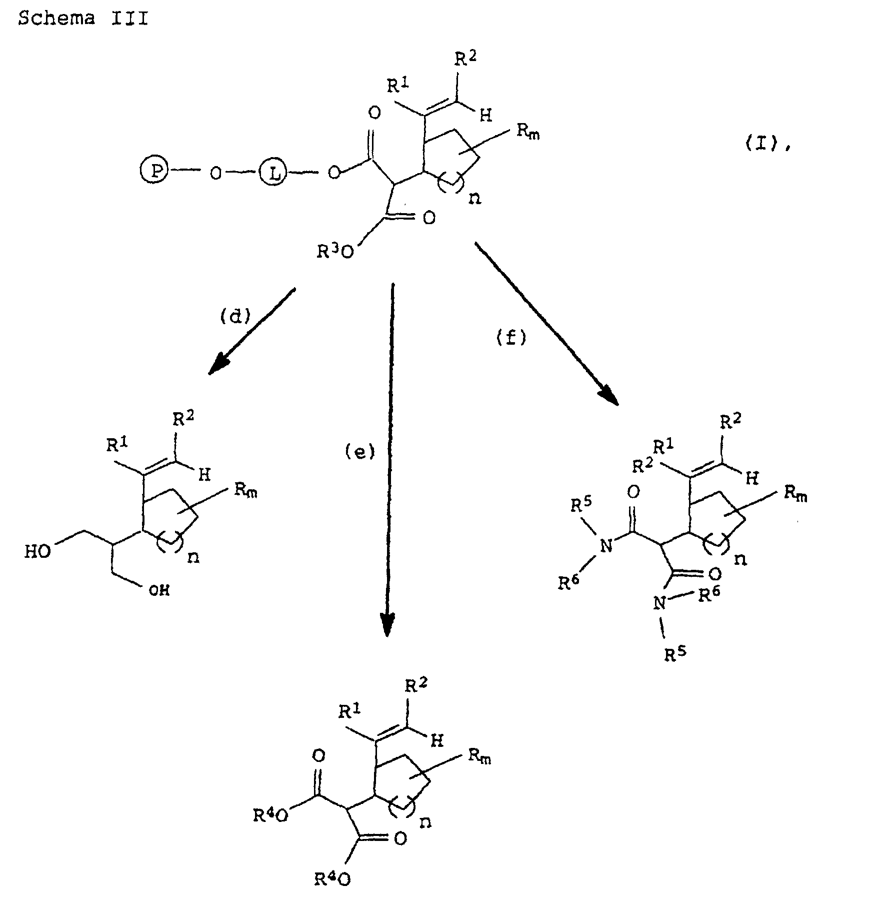

Reaktion (e) wird zur Umesterung geeigneterweise in einem geeigneten Lösungsmittel in einem Temperaturbereich von +40 bis +130°C, bevorzugt +60 bis +100°C in Gegenwart von Ti(OR⁷)₄, wobei R⁷ verzweigte oder unverzweigte C₁-C₆-Alkylketten bedeutet, bevorzugt Ti(OEt)₄ durchgeführt. Als Esterkomponente kommen beliebige Ester in Frage. R⁴ hat folgende Bedeutung:
- Wasserstoff
- C₁-C₁₀-Alkyl, wie beispielsweise Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2Ethylbutyl, 1,21,2-Trimethylpropyl, 1,2,2-Trimethylpropyl, 1-Ethyl-1-methylpropyl, 1-Ethyl-2-methylpropyl, n-Heptyl, n-Octyl, n-Nonyl oder n-Decyl, gegebenenfalls substituiert durch einen gegebenenfalls substituierten aromatischen oder heteroaromatischen Rest mit 3 bis 10 Kohlenstoffatomen. Als Heteroatome können ein oder mehrere Stickstoff-, Schwefel- und/oder Sauerstoffatome im Ring enthalten sein.
- C₃-C₈-Cycloalkyl, wie beispielsweise Cyclopropyl, Cyclobutyl, Cycloheptyl, Cyclohexyl, Cycloheptyl oder Cyclooctyl.
- Aryl wie Phenyl oder Naphthyl, die gegebenenfalls substituiert mit Halogen wie Fluor, Chlor oder Brom, Cyano, Nitro, Amino, Hydroxy oder Thio sein können.

Als Lösungsmittel sind beliebige aprotische Lösungsmittel wie CH₂Cl₂, Toluol, THF oder deren Gemische geeignet.

Soll die freie Säure in der Reaktion (e) entstehen, so wird die Verbindung I vorzugsweise unter Basenkatalyse vom Träger gespalten. Als Basen sind beliebige Basen geeignet, beispielsweise NaOH, LiOH oder KOH. Die Reaktion wird bevorzugt unter Rückfluß in Gegenwart eines mit Wasser mischbaren Lösungsmittels wie Dimethoxyethan, THF, EtOH oder MeOH durchgeführt. Als Temperaturbereich ist 60 bis 130°C, bevorzugt 95 bis 110°C, geeignet.

Die Aminolyse (Reaktion (f)) erfolgt in Gegenwart eines geeigneten Amins der Formel NHR⁵R⁶ in einem Temperaturbereich von 60 bis 130°C, bevorzugt 70 bis 90°C, gegebenenfalls in einem aprotischen Lösungsmittel wie Toluol.

R⁵ und R⁶ haben unabhängig voneinander folgende Bedeutung:
- wasserstoff
- C₁-C₁₀-Alkyl, wie beispielsweise Methyl, Ethyl, n-Propyl, n-Butyl, 1-Methylpropyl, 1-Methylethyl, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimechylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2Ethylbutyl, 1,21,2-Trimethylpropyl, 1,2,2-Trimethylpropyl, 1-Ethyl-1-methylpropyl, 1-Ethyl-2-methylpropyl, n-Heptyl, n-Octyl, n-Nonyl oder n-Decyl, gegebenenfalls substituiert durch einen gegebenenfalls substituierten aromatischen oder heteroaromatischen Rest mit 3 bis 10 Kohlenstoffatomen. Als Heteroatome können ein oder mehrere Stickstoff-, Schwefel- und/oder Sauerstoffatome im Ring enthalten sein.
- C₃-C₈-Cycloalkyl, wie beispielsweise Cyclopropyl, Cyclobutyl, Cycloheptyl, Cyclohexyl, Cycloheptyl oder Cyclooctyl.
- Aryl wie Phenyl oder Naphthyl, die gegebenenfalls substituiert mit Halogen wie Fluor, Chlor oder Brom, Cyano, Nitro, Amino, Hydroxy oder Thio sein können.

Das erfindungsgemäße Verfahren eignet sich sehr gut zur Erzeugung einer großen Zahl strukturell vielfältiger Verbindungen der Formeln I, IV und VI, da die Substituenten R, R¹ bis R⁷ unabhängig voneinander auf einfache Weise breit variierbar sind.

Im Vergleich zu Reaktionen in Lösung weisen die Umsetzungen am polymeren Träger große Vorteile auf. So findet man in den Produkten erheblich weniger Verunreinigungen, so daß eine chromatographische Auftrennung nicht erforderlich ist. Insbesondere tritt die in Lösung häufig beobachtete Doppelbindungsisomerisierung der primär gebildeten Alkyliden-1,3-dicarbonyl-Verbindung kaum auf; außerdem stört die als Nebenreaktion ablaufende intramolekulare Carbonyl-En-Reaktion der Aldehyde nicht, da die entsprechenden Alkohole beim Waschen der polymergebundenen Produkte entfernt werden. Die guten Ausbeuten, die hohe Reinheit der abgespaltenen Produkte und die einfache Reaktionsführung des erfindungsgemäßen Verfahrens machen seine Anwendung im Rahmen der kombinatorischen Synthese sehr attraktiv. Besonders vorteilhaft bei diesem Verfahren ist beispielsweise, daß auf die Verwendung teurer Polymere verzichtet werden kann, da ein kostengünstiger Linker zur Funktionalisierung an beliebige feste Phasen angebunden werden kann.

Das Verfahren eignet sich auch besonders gut zur Herstellung definierter Gemische von Cycloalkylderivaten der Formel I. Dazu geht man nicht von einer Einzelsubstanz aus, die an die feste Phase gebunden wird, sondern bindet ein Gemisch, bevorzugt ein nach Stöchiometrie und Substanzen bekanntes Gemisch, an die feste Phase.

Der festphasengebundene Reaktionspartner wird dann gemäß dem beschriebenen Verfahren mit dem anderen Reaktionspartner umgesetzt und anschließend ggf. zyklisiert.

So lassen sich beispielsweise ausgehend von Malonsäuremonoesterchloriden, 8 Aldehyden und anschließender Abspaltung durch Umesterung mit 4 verschiedenen Estern oder Aminolyse mit 4 verschiedenen Aminen gemäß der Methode von Furka, A.; Sebestyen, F.; Asgedom, M.; Dibo, G. 1988, Abstr. 14th Int. Congr. Biochem, Prague, Czechoslovakia, Vol. 5, p. 47. Abstr. 10th Int. Symp. Med. Chem., Budapest, Hungary, p. 288. Furka, A.; Sebestyen, F.; Asgedom, M.; Dibo, G. General Method for Rapid Synthesis of Multicomponente Peptide Mixtures. Int. J. Pept. Protein Res. 1991, 37, 487-493; 64 Cycloalkylderivate erhalten.

Der Vorteil dieser Festphasensynthese liegt in der schnellen Erzeugung einer Vielzahl von einzelnen Verbindungen, die anschließend auf ihre Wirksamkeit in Testsystemen untersucht werden können.

Dazu können die Substanzgemische entweder vorher aufgetrennt werden oder direkt in der Form als Gemisch eingesetzt werden. Im zweiten Fall erfolgt eine Identifizierung eines potentiellen Wirkstoffes nach der Testung.

Ein weiterer Gegenstand der Erfindung ist die Verwendung des erfindungsgemäßen Herstellverfahrens für gebundene oder freie Cycloalkylderivate der Formeln I oder IV zur Generierung von Substanzbibliotheken.

Hierunter ist sowohl die oben beschriebene Erzeugung von Cycloalkylgemischen als auch die Herstellung einer Vielzahl von Einzelsubstanzen der Formeln I, IV oder VI, beispielsweise durch paralleles Ausführen vieler gleichartiger Reaktionen, bei der jeweils ein Reaktionspartner verändert wurde, zu verstehen.

Das parallele Ausführen vieler gleichartiger Reaktionen erlaubt auf schnelle Weise die systematische Variation aller funktionellen Gruppen in den Formeln I, IV oder VI.

Ein weiterer Gegenstand der Erfindung sind trägergebundene Cycloalkylderivate der allgemeinen Formeln I, II oder IV. Diese Verbindungen sind herstellbar, indem man das obengenannte Herstellverfahren ohne eine Abspaltung des erhaltenen Cycloalkyls der Formel I oder der Zwischenprodukte II oder IV von der festen Phase, ausführt.

Dadurch bleiben die Cycloalkyle oder die Zwischenprodukte an die feste Phase gebunden und können als solche leicht in Testverfahren, bevorzugt in in-vitro Testsystemen eingesetzt werden.

Der Vorteil der trägergebundenen Cycloalkylderivate und Zwischenprodukte liegt in ihrer leichten Handhabbarkeit. Beispielsweise können sie leicht aus der Reaktionslösung durch Filtration oder Zentrifugation isoliert werden.

Zusätzlich ist die Identifizierung eines Wirkstoffs erheblich erleichtert, da die trägergebundenen Cycloalkylderivate schon vereinzelt vorliegen und so eine Trennung entfällt.

Die folgenden Beispiele dienen der weiteren Veranschaulichung der Erfindung, ohne sie in irgendeiner Weise einzuschränken.

### Beispiel 1

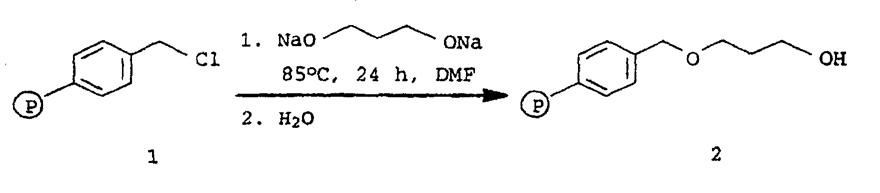

5 g Merrifield-Harz 1 (2,58 mmol Cl/g, 2 % DVB, Acros), gequollen in DMF, wurden mit 26 ml einer 1M Lösung von Dinatrium-1,3-propandiolat in DMF (26 mmol, 2 Äquiv.) versetzt. Die Suspension wurde 24 h bei 85°C erhitzt. Nach Abkühlen auf ca. 60°C erfolgte die Zugabe von 50 ml Wasser und dem gleich Volumen an DMF. Es wurde weitere 20 min gerührt. Anschließend wurde das Linker-modifizierte Harz 2 über eine Fritte abgesaugt, mit DMF/Wasser, DMF, Methanol, Methylenchlorid gewaschen und im Wasserstrahlvakuum bei 55°C für 24 h getrocknet.

Analog wurde die Reaktion mit 1,5-Pentandiol durchgeführt.

Zur Bestimmung der Konzentration der Hydroxygruppen des Linkerverknüpften Harzes 2 wurde dieses mit 3,5-Dinitrobenzoylchlorid in Pyridin umgesetzt, die Stickstoffbestimmung des erhaltenen Esters ergab 0,75 mmol Hydroxygruppen pro Gramm Harz.

### Beispiel 2

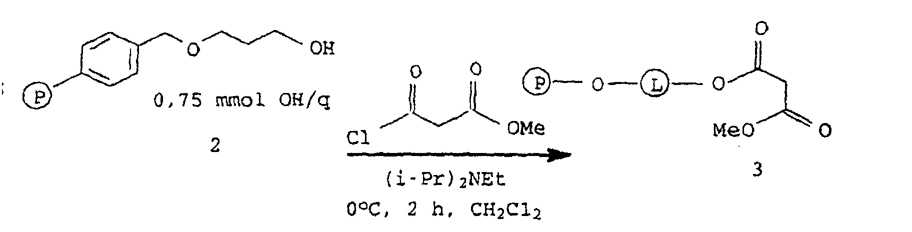

In einer Glasapparatur wird unter Schutzgasatmosphäre eine Suspension des in Methylenchlorid (12 ml) gequollenen Harzes 2 (2,5 g) mit 642 µl Hünig-Base (Diisopropylethylamin; 3,75 mmol, 2 Äquiv.) versetzt und auf 0°C abgekühlt. Unter kräftigem Rühren werden langsam 402 µl Malonsäuremonomethylesterchlorid (3,75 mmol, 2 Äquiv.) hinzugetropft. Nach beendeter Zugabe wird bei gleicher Temperatur 2 h gerührt und anschließend noch 1 h bei 20°C. Das Harz 3 wird abfiltriert, mit Methanol und Methylenchlorid gewaschen und bei 55°C im Wasserstrahlvakuum getrocknet.

Analog wurde die Reaktion mit folgendem Harz durchgeführt.

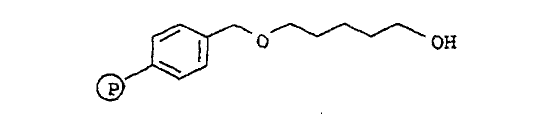

### Beispiel 3

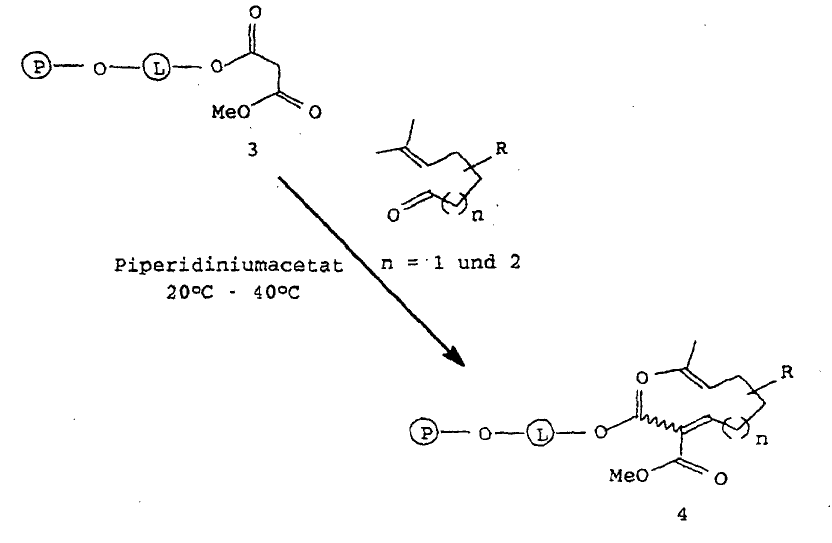

In einer Glasapparatur wird unter Schutzgasatmosphäre das in Methylenchlorid (6 ml) gequollene malonatfunktionalisierte Polymer 3 (1 g) mit den entsprechenden Aldehyden (3 Äquiv.) versetzt. Es wird 30 min bei 20°C gerührt. Es erfolgt nacheinander die Zugabe von 8,6 µl 99,9 %igem Eisessig (0,15 mmol, 0,2 Äquiv., bezogen auf polymeres Malonat) und 14,8 µl frischdestilliertem Piperidin (0,15 mmol, 0,2 Äquiv., bezogen auf polymeres Malonat). Man läßt die Reaktionen 2 h reagieren und gibt anschließend nochmals die gleiche Menge an Katalysator hinzu und rührt eine weitere Stunde. Nach beendeter Reaktion wird das Harz 4 abfiltriert und mit Methylenchlorid gewaschen und nicht trocken gesaugt (Feuchtigkeitsausschluß!).

Bei der Verwendung α-substituierter Aldehyde erfolgt zusätzlich die Zugabe von frisch ausgeheiztem Natriumsulfat (ca. 5 Spatelspitzen bei Verwendung von 1 g Harz). Die Temperatur wird bevorzugt auf 40°C erhöht. Die Reaktionsmischung wird bei sonst identischer Reaktionsführung refluxiert.

Die Reaktionen (d.h. das Abreagieren der Aldehyde) können mit 1-Dodecen als internen Standard per GC verfolgt werden.

### Beispiel 4

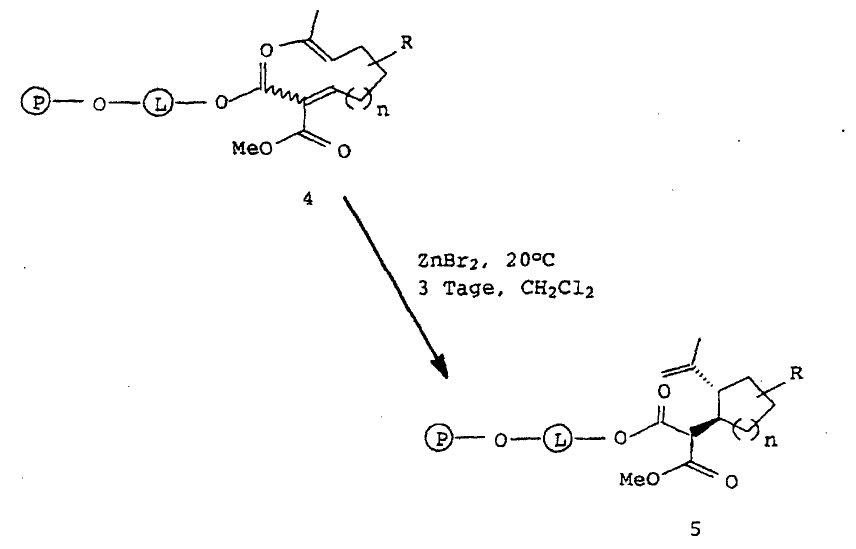

In einer Glasapparatur wird unter Schutzatmosphäre das in Methylenchlorid (7 ml) gequollene Harz 4 (ca. 1 g), siehe oben) mit 202 mg frisch ausgeheiztem Zinkbromid (0,83 mmol, 1,1 Äquiv.) versetzt. Die Suspension wird 3 Tage bei 20°C gerührt. Anschließend wird mit Methanol das suspendierte Zinkbromid in Lösung gebracht. Das Harz 5 wird abfiltriert, mit Methanol und Methylenchlorid gewaschen und bei 55°C 24 h im Wasserstrahlvakuum getrocknet.

### Beispiel 5

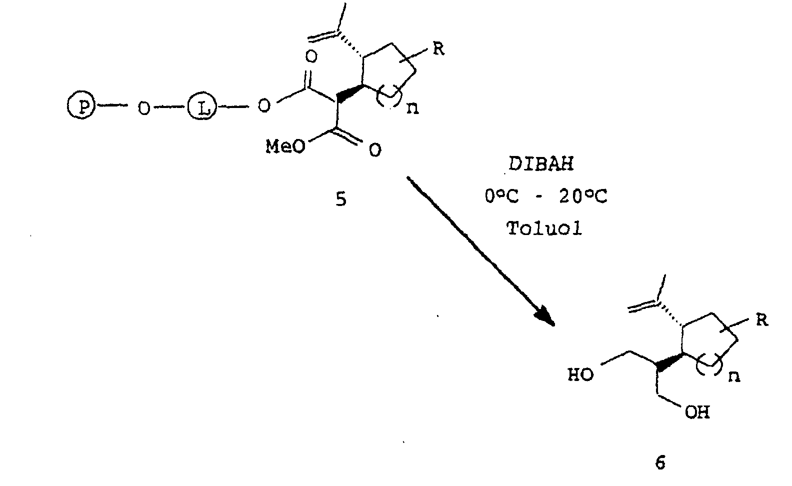

In einer Glasapparatur wird unter Schutzgasatmosphäre das in Toluol (5 ml) gequollene Harz 5 (1 g) auf 0°C abgekühlt und tropfenweise mit 10 ml einer 1,2 molaren Diisobutylaluminiumhydrid-Lösung (DIBAH) in Toluol (12 mmol, 4 Äquiv./Esterfunktion) versetzt. Nach beendeter Zugabe läßt man über Nacht auf 20°C kommen. Zur Zerstörung von überschüssigem DIBAH wird die Reaktionsmischung vorsichtig unter Eiskühlung mit Methanol versetzt (Reaktionsmischung kann zu einem gel-artigen Feststoff werden) . Anschließend wird mit dem gleichen Volumen (bezogen auf gesamte Reaktionsmischung) einer Kalium-natrium-tartrat-Lösung kräftig geschüttelt und die organische Phase abgetrennt. Das Harz sammelt sich hauptsächlich an der Phasengrenze und muß nicht in einem separaten Arbeitsschritt abgetrennt werden. Die wäßrige Phase wird solange mit tert. -Butyl -methyl -ether extrahiert, bis kein Diol mehr nachzuweisen ist. Die vereinigten organischen Phasen werden nach Waschen mit NaCl-Lösung und Trocknen über wasserfreies Na₂SO₄ im Vakuum eingeengt und der Rückstand im Ölpumpenvakuum getrocknet.

### Beispiel 6

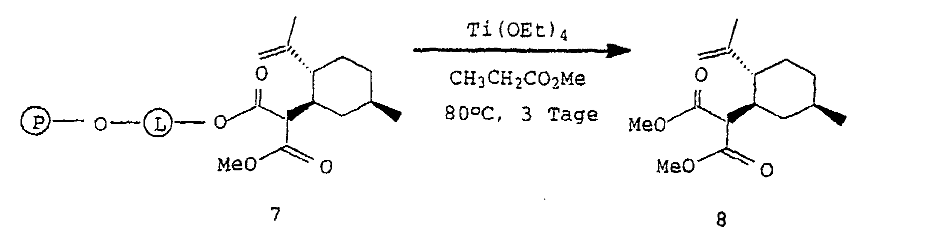

In einer Glasapparatur wird unter Schutzatmosphäre 1 g des Harzes 7 in 10 ml trockenem Propionsäuremethylester suspendiert. Nach Zugabe von 157 µl Ti(OEt)₄ (0,75 mmol, 1 Äquiv.) wird die Reaktionsmischung 3 Tage unter Rückfluß erhitzt. Nach Abkühlen auf 20°C wird die Reaktionsmischung mit ca. 20 ml 2 N HCl versetzt und kräftig geschüttelt. Das Harz sammelt sich hauptsächlich an der Phasengrenze und muß nicht in einem separaten Arbeitsschritt abgetrennt werden. Nach Trennen der Phasen wird die wäßrige Phase mehrmals mit tert.-Butyl-methylether extrahiert. Die gesammelten organischen Phasen werden mit NaHCO₃ gewaschen , über wasserfreies Na₂SO₄ getrocknet und im Vakuum bis zur Trockene eingedampft. Es werden 82 mg der Verbindung 8 erhalten (41 % der Ausbeute, bezogen auf Konzentration an freien OH-Gruppen im spacermodifizierten Polymer).

### Beispiel 7

Die in den Beispielen 3 bis 6 dargestellte getrennt durchgeführte Reaktionssequenz läßt sich auch in einem Reaktionskessel ohne Isolierung der einzelnen Zwischenprodukte durchführen,

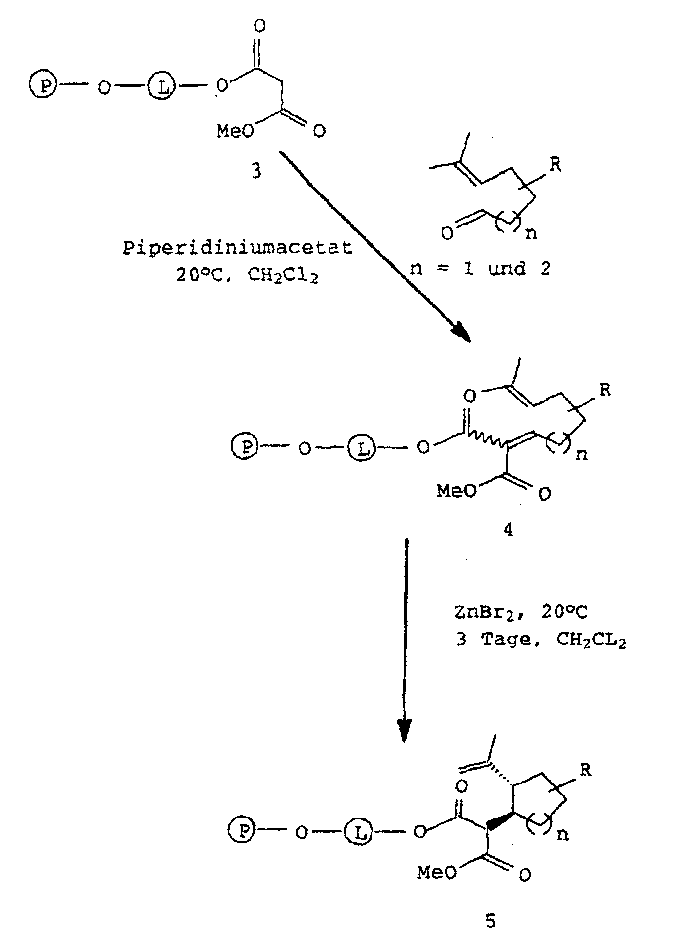

In einer Glasapparatur wird unter Schutzatmosphäre das in Methylchlorid (6 ml) gequollene malonatfunktionalisierte Polymer 3 (1 g) mit den entsprechenden Aldehyden (3 Äquiv.) versetzt. Es wird 30 min bei 20°C gerührt. Es erfolgt nacheinander die Zugabe von 8,6 µl 99,9 %igem Eisessig (0,15 mmol, 0,2 Äquiv., bezogen auf polymeres Malonat) und 14,8 µl frischdestilliertem Piperidin (0,15 mmol, o,2 Äquiv., bezogen auf polymeres Malonat). Man läßt 2 h reagieren und gibt anschließend nochmals die gleiche Menge an Katalysator hinzu und rührt eine weitere Stunde. Es folgt die Zugabe von 202 mg frisch ausgeheiztem Zinkbromid (0,83 mmol, 1,1 Äquiv.) und 1 ml Methylenchlorid. Die Suspension wird 3 Tage bei 20°C gerührt. Anschließend wird mit Methanol das suspendierte Zinkbromid in Lösung gebracht. Das Harz 5 wird abfiltriert, mit Methanol und Methylenchlorid gewaschen und bei 55°C 24 h im Wasserstrahlvakuum getrocknet.

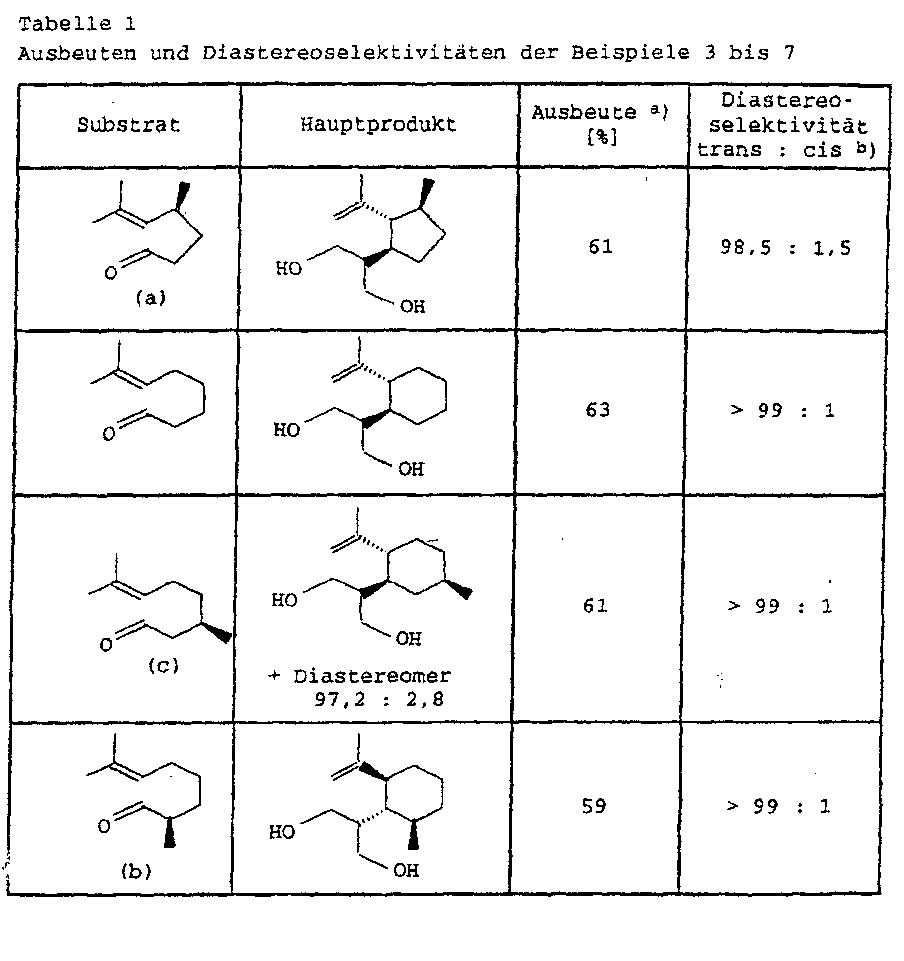

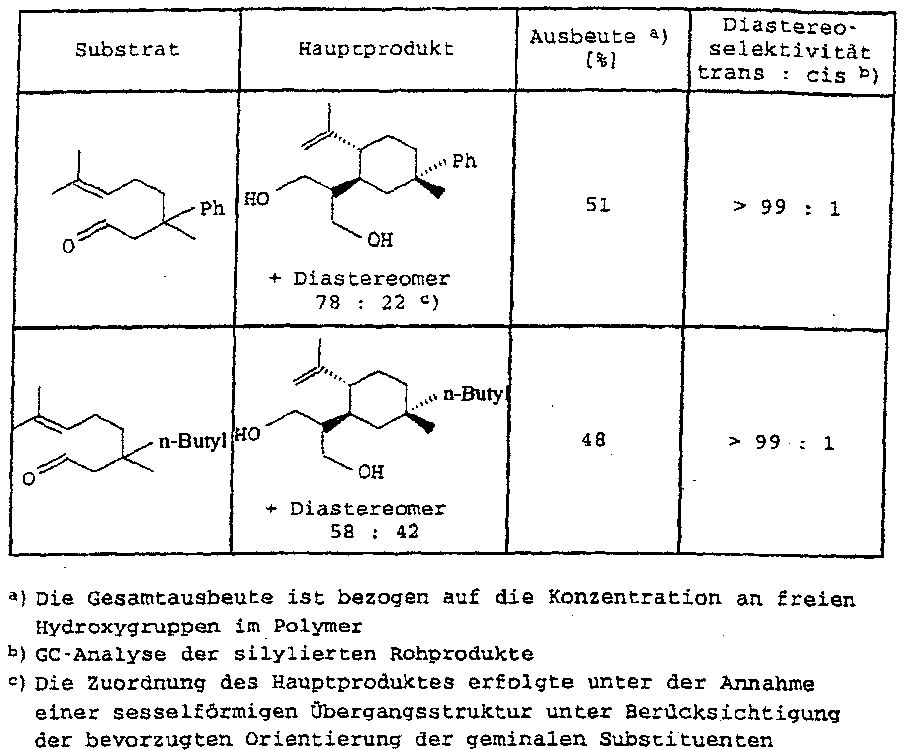

Sowohl in dieser Eintopfvariante als auch in den Beispielen 3 bis 6 lassen sich die Produkte in guten Ausbeuten und in hoher Reinheit sowie exzellenter einfacher und induzierter Diastereoelektrizität herstellen. Die Ausbeuten, bezogen auf freie Hydroxygruppen im Polymer betrug 41 bis 61 %. Die Reinheit der abgespaltenen Produkte lag üblicherweise bei 90 %. Die Zyklisierungsreaktion (En-Reaktion) wird bezüglich der einfachen und induzierten Diastereoselektivität nicht durch die Bindung an das Polymer beeinflußt (siehe Tabelle 1). Es wurden trans-1,2-disubstituierte Cyclohexane mit einer einfachen Diastereoselektivität von ds > 99:1 und die entsprechenden trans-1,2-disubstituierten Cyclopentane mit ds = 98,5:1,5 erhalten.

Die induzierte Diastereoelektrizität betrug bei den α-monosubstituierten Aldehyden a und b jeweils ds > 99:1, während mit dem β-monosubstituierten Aldehyd c eine induzierte Diastereoselektivität von ds = 96,9:3,1 gefunden wurde. Spektroskopische Daten für 3 Beispiele 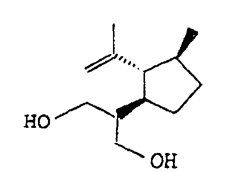
- ¹H-NMR: (CDCl₃, 200 MHz): δ = 0.89 (d, J = 6 Hz; 3 H, 3' -CH₃) , 0.96 - 1.89 (m; 7 H, übrige Protonen), 1.68 (mc; 3 H, 2" -CH₃), 1.98 (mc; 1 H), 2.38 (s br; 2 H, OH, mit D₂O austauschbar), 3.53 - 3.93 (m; 4 H, 1-H₂, 3-H₂), 4.82 (mc; 2 H, 1" -H₂) .
- ¹³C-NMR: (CDCl₃, 20 MHz): δ = 18.21 und 18.91 (C-3" und 3'-CH₃), 27.51 (C-5'). 32.83 (C-4'), 38.68 (C-3'), 41.43 (C-1'), 45.64 (C-2), 60.42 (C-2'), 63.53 und 64.58 (C-1 und C-3), 112,44 (C-1" ) , 146.75 (C-2" ) .
- MS (70 eV):: m/e = 198 (1 %, M⁺), 183 (2 %, M-CH₃), 180 (9 %, M-H₂O), 165 (10 %, 180-CH₃), 123 (100 %, C₉H₁₅) , 109 (41 %), 107 (42 %, C₈H₁₁) , 93 (36 %, C₇H₉), 83 (45 %, C₆H₁₁) , 82 (35 %), 81 (80 %, C₆H₉) , 79 (37 %, C₆H₇), 67 (54 %, C₅H₇) , 55 (58 %, C₄H₇), 43 (34 %, C₃H₇), 41 (65 %, C₃H₅) .

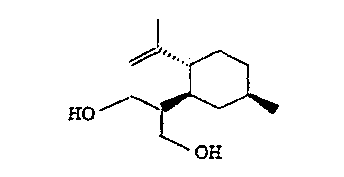

- ¹H-NMR: (CDCl₃, 200 MHz): δ = 0.64 (q, J = 12 Hz; 1 H, 6' -Hₐₓ) , 0.87 (d, J = 6.5 Hz; 3 H, 5'-CH₃), 0.91 (d q, J = 4, 12 Hz; 1 H, 3'-Hₐₓ oder 4'-Hₐₓ), 1.14 - 1.82 (m; 6 H, übrige Protonen), 1.66 (mc; 3 H, 2''-CH₃), 1.84 - 2.06 (m; 2 H), 2.17 (mc; 1 H, OH, mit D₂O austauschbar), 2.37 (mc; 1 H, OH, mit D₂O austauschbar), 3.56 - 3.76 (m; 2 H, 1-H₂ oder 3-H₂, nach D₂O-Austausch Signalverschärfung), 3.78 - 4.00 (m; 2 H, 1-H₂ oder 3-H₂, nach D₂O-Austausch Signalverschärfung), 4.78 (s br; 2 H, 1" -H₂) .
- ¹³C-NMR: (CDCl₃, 50 MHz): δ = 18.43 (C-3" ) , 22.72 (5' -CH₃) , 32.65 (C-3'), 32.97 (C-5'), 34.93 (C-4' ), 35.96 (C-6'), 39.60 (C-1'), 42.96 (C-2), 49.14 (C-2'), 62.96 und 66.73 (C-3), 111.76 (C-1''), 148.50 (C-2").
- MS (70 eV):: m/e = 212 (0.3 %, M⁺, HA), 194 (8 %, M-H₂O) , 151 (17 %, M-C₂H₅O₂) , 137 (67 %, C₁₀H₁₇) , 109 (43 %), 107 (41 %), 95 (79 %, C₇H₁₁) , 93 (49 %), 81 (100 %, C₆H₉, 69 (51 %, C₅H₉), 67 (51 %, C₅H₇), 55 (51 %, C₄H₇) ,41 (60%, C₃H₅) .

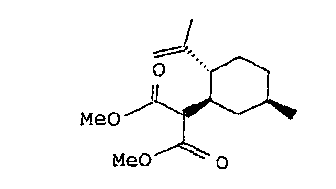

- ¹H- NMR: (CDCl₃, 200 MHz): δ = 0.91 (d, J = 6,5 Hz; 3 H, 5'-CH₃), 0.95 (d q, J = 3.5, 12 Hz; 1 H, 4'-Hₐₓ), 1.11 (q, J = 11.5 Hz; 1 H, 6' -Hₐₓ) , 1.24 - 1.57 (m; 2 H, 3' -Hₐₓ, 5'-Hₐₓ), 1.65 (mc; 3 H, 2'' -CH₃) , 1.57 - 1.94 (m; 3 H, 3'-H_{eq'} 4'-H_{eq}, 6' H_{eq}) , 2.05 (d t, J = 3.0, 11.5 Hz; 1 H, 2'H), 2.13 (t t, J = 3.5, 11.5 Hz; 1 H, 1'H) , 3.56 (d, J. = 3.5 Hz; 1 H, 2-H), 3.73 (s; 6 H, OCH₃). 4.74 (mc; 1 H, 1" :H) , 4.79 (m; 1 H, 1' 'H) .
- ¹³C -NMR: (CDCl₃, 50 MHz) : δ = 18.96 (C-3") , 22, 54 (5' - CH₃) , 32.35 (C-3'), 32.73 (C-5'), 34.68 (C-4'), 36.59 (C-6'), 39.88 (C-1'), 48.68 (C-2'), 51.80 und 52.22 (OCH₃), 53.22 (C-2), 112.43 (C-1"), 147.52 (C-2"), 169.03 und 170.11 (C-1 und C-3).
- MS (70 eV):: m/e = 268 (4 %, M⁺), 250 (1%, M-H₂O), 237 (3 %, M-CH₃O), 236 (3 %, M-CH₃OH) , 209 (3 %, M-C₂H₃O₂) , 208 (7 %, M-C₂H₄O₂), 137 (19 %, C₁₀H₁₇) , 136 (100 %, C₁₀H₁₆, McL, HA), 133 (29 %), 132 (16 %, C₅H₈O₄) , 121 (35 %, C₉H₁₃) , 107 (41 %, C₈H₁₁) , 94 (14 %, C₇H₁₀, RDA), 93 (34 %, C₇H₉) , 79 (21 %, C₆J₇) , 59 (10 %, C₂H₃O₂).

## Patentansprüche

1. Verfahren zur Herstellung von Cycloalkylderivaten der Formel I 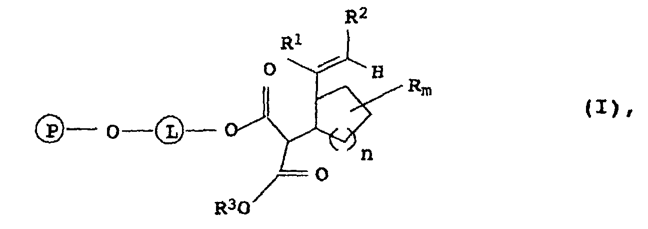 in der die Variablen und Substituenten folgende Bedeutung haben:
(P) eine feste Phase ausgewählt aus der Gruppe Keramik, Glas, Latex, quervernetzte Polystyrole, Polyacrylamide, Silicagele, Cellulosepartikel, Harze, Gold oder colloidale Metallpartikel,
(L) einen Linker mit 2 bis 12 Kohlenstoffatomen oder der Struktur -CH₂-CH₂(-O-CH₂-CH₂-)₁₋₁₀₀-,
R¹ Wasserstoff oder einen niedermolekularen organischen Rest,
R² Wasserstoff oder gegebenenfalls substituiertes Alkyl, Alkenyl, Alkinyl oder Cyclkoalkyl oder
R¹ und R² zusammen einen gegebenenfalls substituierten 4 bis 8-gliedrigen Ring
R³ ggf. substit. C₁-C₁₀-Alkyl, C₃-C₈-Cycloalkyl oder Aryl,
R einen niedermolekularen organischen Rest oder zwei benachbarte Reste R zusammen einen gegebenenfalls substituierten carbo- oder heterocyclischen Ring bilden
n = 0 bis 4
m = 0 bis n + 2,
**dadurch gekennzeichnet, daß** man eine Verbindung der Formel II 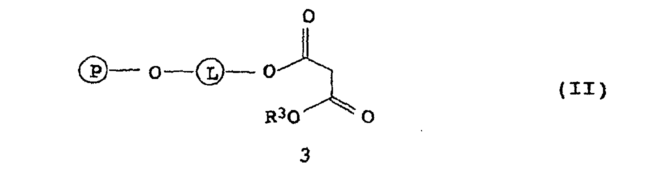 mit Aldehyden der Formel III 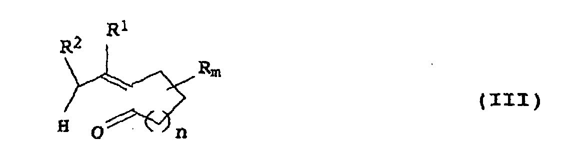 in Gegenwart einer Base zu Verbindungen der Formel IV 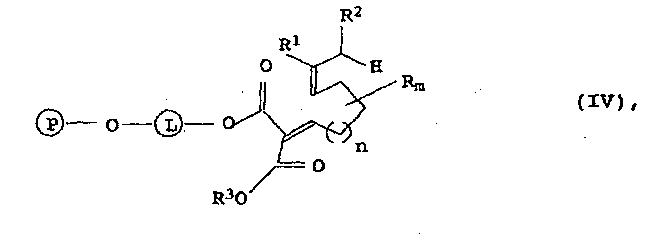 umsetzt, und das erhaltene Produkt IV in Gegenwart einer Lewis-Säure zyklisiert.

2. Verbindungen der Formel I gemäß Anspruch 1.

3. Verfahren zur Herstellung von Verbindungen der Formel II gemäß Anspruch I, **dadurch gekennzeichnet, daß** man Verbindungen der Formel VII 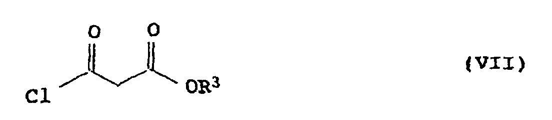 an eine funktionalisierte feste Phase der Formel V
Ⓟ―O―Ⓛ―OH (V)
wobei
(P) eine feste Phase ausgewählt aus der Gruppe Keramik, Glas, Latex, quervernetzte Polystyrole, Polyacrylamide, Silicagele, Cellulosepartikel, Harze, Gold oder colloidale Metallpartikel und
(L) einen Linker mit 2 bis 12 Kohlenstoffatomen oder der Struktur -(-O-CH₂-CH₂-)₁₋₁₀₀- bedeutet,
in Gegenwart einer Base bindet.

4. Verbindung der allgemeinen Formel V gemäß Anspruch 3
Ⓟ― O―Ⓛ― OH (V)
wobei
(P) eine feste Phase ausgewählt aus der Gruppe Keramik, Glas, Latex, quervernetzte Polystyrole, Polyacrylamide, Silicagele, Cellulosepartikel, Harze, Gold oder colloidale Metallpartikel und
(L) einen Linker mit 2 bis 12 Kohlenstoffatomen bedeutet.

5. Verbindungen der Formel II gemäß Anspruch 1.

6. Verbindungen der Formel IV gemäß Anspruch 1.

7. Verfahren gemäß Anspruch 1 zur Herstellung von Substanzbibliotheken.

8. Verwendung von Verbindungen der Formel I gemäß Anspruch 1 zur Herstellung von Substanzbibliotheken der allgemeinen Formel VI 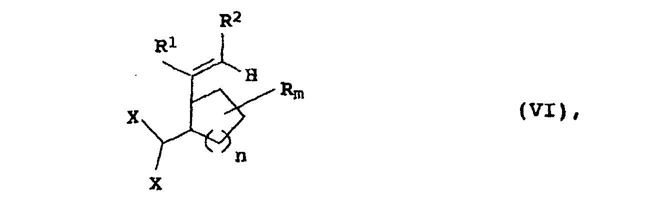 wobei
X -COOR⁴, -CONR⁵R⁶, -CH₂OH bedeutet und
R⁴, R⁵ und R⁶ unabhängig voneinander Wasserstoff, C₁-C₁₀-Alkyl-, gegebenenfalls substituiert durch einen gegebenenfalls substituierten aromatischen oder heteroaromatischen Rest, C₃-C₈-Cycloalkyl-, ggf. subst. Aryl-, R¹, R², Rm, n, m die in Anspruch 1 angegebene Bedeutung besitzen **dadurch gekennzeichnet, daß** Verbindungen der Formel I unter Reduktion, Umesterung, Amidierung oder Basenkatalyse von der festen Phase abgespalten werden.

9. Verwendung von Verbindungen der Formel IV gemäß Anspruch 6 zur Herstellung von Substanzbibliotheken.

10. Verwendung von Verbindungen der Formel II gemäß Anspruch 5 als CH-acide Komponente in C-C-Verknüpfungsreaktionen.

11. Verwendung der Verbindungen v als feste Phase gemäß Anspruch 4, als funktionalisierter Träger für organische Synthesen, für chromatographische Verfahren, zur Immobilisierung von Wirkstoffen oder Antigenen.

## Claims

1. A process for preparing cycloalkyl derivatives of the formula I 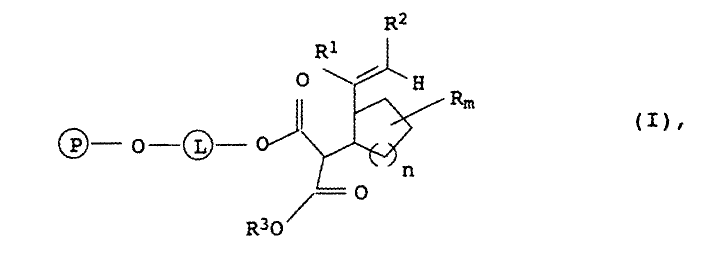 in which the variables and substituents have the following meanings:
(P) a solid phase selected from the group consisting of ceramic, glass, latex, crosslinked polystyrenes, polyacrylamides, silica gels, cellulose particles, resins, gold and colloidal metal particles,
(L) a linker with 2 to 12 carbon atoms or the structure - CH₂-CH₂ (-O-CH₂-CH₂-) ₁₋₁₀₀-,
R¹ hydrogen or a low molecular weight organic radical,
R² hydrogen or unsubstituted or substituted alkyl, alkenyl, alkynyl or cycloalkyl or
R¹ and R² together an unsubstituted or substituted 4- to 8-membered ring
R³ unsubstituted or substituted C₁-C₁₀-alkyl, C₃-C₈-cycloalkyl or aryl,
R a low molecular weight organic radical or two adjacent R radicals together form an unsubstituted or substituted carbo- or heterocyclic ring
n = 0 to 4
m = 0 to n + 2,
which comprises reacting a compound of the formula II 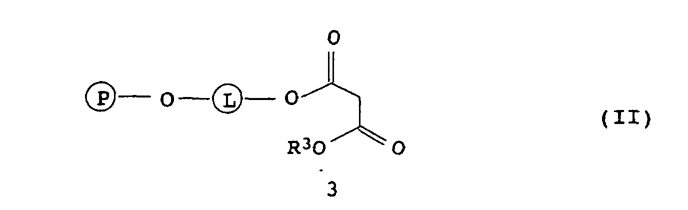 with aldehydes of the formula III 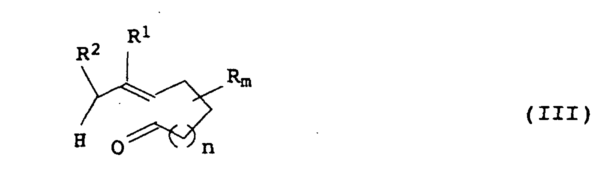 in the presence of a base to give compounds of the formula IV 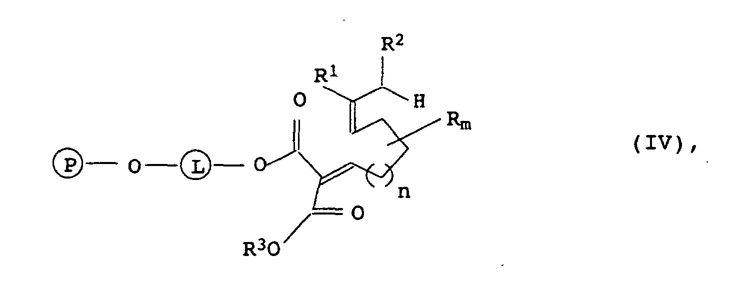 and cyclizing the resulting product IV in the presence of a Lewis acid.

2. A compound of the formula I as set forth in claim 1.

3. A process for preparing compounds of the formula II as set forth in claim 1, which comprises linking compounds of the formula VII 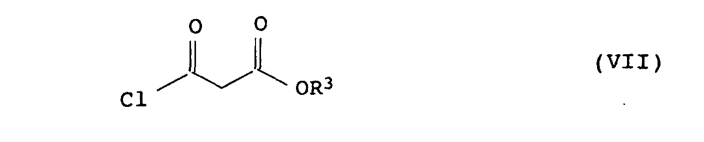 to a functionalized solid phase of the formula V
Ⓟ―O―Ⓛ――OH (V)
where
(P) is a solid phase selected from the group consisting of ceramic, glass, latex, crosslinked polystyrenes, polyacrylamides, silica gels, cellulose particles, resins, gold and colloidal metal particles, and
(L) is a linker with 2 to 12 carbon atoms or the structure - CH₂(̵O-CH₂)̵₁₋₁₀₀,
in the presence of a base.

4. A compound of the general formula V as set forth in claim 3
Ⓟ― O―Ⓛ― OH (V)
where
(P) is a solid phase selected from the group consisting of ceramic, glass, latex, crosslinked polystyrenes, polyacrylamides, silica gels, cellulose particles, resins, gold and colloidal metal particles, and
(L) is a linker with 2 to 12 carbon atoms.

5. A compound of the formula II as set forth in claim 1.

6. A compound of the formula IV as set forth in claim 1.

7. A process as claimed in claim 1 for producing substance libraries.

8. The use of compounds of the formula I as set forth in claim 1 for producing substance libraries of the general formula VI 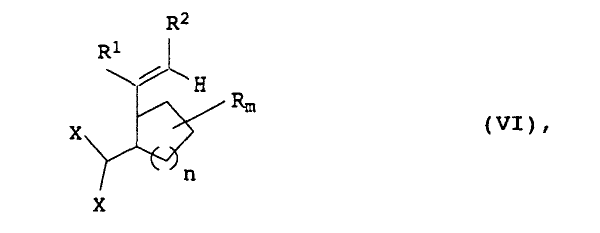 where
X is -COOR⁴, -CONR⁵R⁶ or -CH₂OH, and
R⁴, R⁵ and R⁶ are, independently of one another, hydrogen, C₁-C₁₀-alkyl, unsubstituted or substituted by an unsubstituted or substituted aromatic or heteroaromatic radical, C₃-C₈-cycloalkyl, unsubstituted or substituted aryl, R¹, R², Rm, n, m have the meanings stated in claim 1, wherein compounds of the formula I are cleaved off the solid phase by reduction, transesterification, amidation or base catalysis.

9. The use of compounds of the formula IV as claimed in claim 6 for producing substance libraries.

10. The use of compounds of the formula II as claimed in claim 5 as carbon acid component in C-C linkage reactions.

11. The use of the compounds V as solid phase as claimed in claim 4 as functionalized support for organic syntheses, for chromatographic methods, and for immobilizing active substances or antigens.

## Revendications

1. Procédé pour la préparation de dérivés cycloalkyliques de formule I : 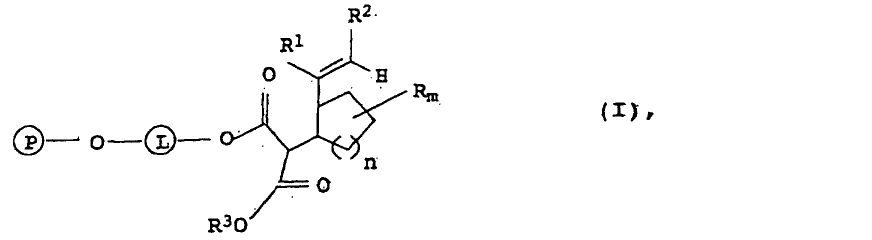 dans laquelle les symboles et indices ont les significations suivantes :
(P) une phase solide choisie dans le groupe consistant en les matières céramiques, le verre, les latex, les polystyrènes réticulés, les polyacrylamides, les gels de silice, les particules de cellulose, les résines, l'or et les particules de métaux colloïdaux,
(L) un chaînon de 2 à 12 atomes de carbone ou de structure
- CH₂-CH₂(-O-CH₂-CH2-)_{1-100-,}
R¹ l'hydrogène ou un radical organique à bas poids moléculaire,
R² l'hydrogène ou un groupe alkyle, alcényle, alcynyle ou cycloalkyle éventuellement substitué, ou bien,
R¹ et R² forment ensemble un cycle de 4 à 8 chaînons éventuellement substitué,
R³ un groupe alkyle en C1-C10, cycloalkyle en C3-C8 ou aryle éventuellement substitué,
R un radical organique à bas poids moléculaire, ou bien deux groupes R voisins forment ensemble un noyau carbo- ou hétéro-cyclique éventuellement substitué,
n égal 0 à 4,
m égal 0 à n+2
**caractérisé en ce que** l'on fait réagir un composé de formule II 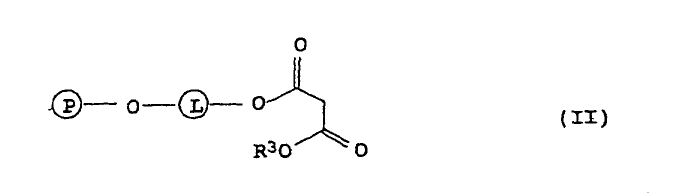 avec des aldéhydes de formule III 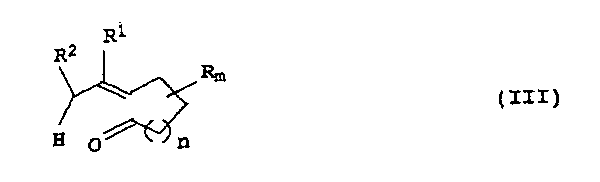 en présence d'une base, ce qui donne des composés de formule IV 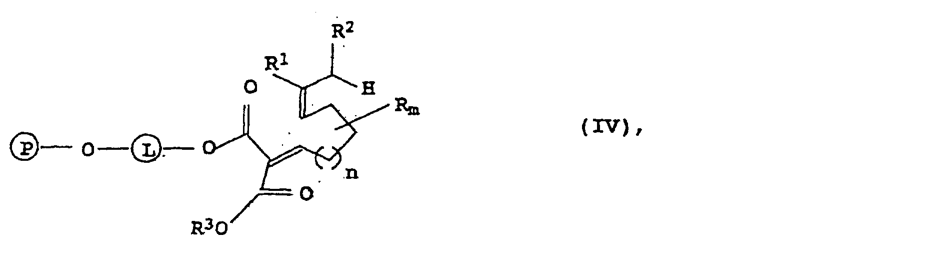 qu'on cyclise en présence d'un acide de Lewis.

2. Composés de formule I selon la revendication 1.

3. Procédé pour la préparation des composés de formule II selon la revendication 1, **caractérisé en ce que** l'on fixe des composés de formule VII 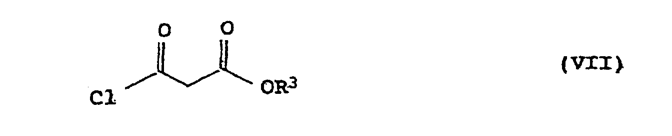 sur une phase solide fonctionnalisée de formule V
Ⓟ―O―Ⓛ―OH (V)
(P) représentant une phase solide choisie dans le groupe consistant en les matières céramiques, le verre, les latex, les polystyrènes réticulés, les polyacrylamides, les gels de silice, les particules de cellulose, les résines, l'or ou les particules de métaux colloïdaux et
(L) représente un chaînon de 2 à 12 atomes de carbone ou de structure - (O-CH₂CH₂)_{1 à 100}-_{,}
en présence d'une base.

4. Composés de formule générale V selon la revendication 3
Ⓟ―O―Ⓛ-OH (V)
dans laquelle
(P) représente une phase solide choisie dans le groupe consistant en les matières céramiques, le verre, les latex, les polystyrènes réticulés, les polyacrylamides, les gels de silice, les particules de cellulose, les résines, l'or ou les particules de métaux colloïdaux et
(L) représente un chaînon de 2 à 12 atomes de carbone.

5. Composés de formule II selon la revendication 1.

6. Composés de formule IV selon la revendication 1.

7. Procédé selon la revendication 1 pour la fabrication de collections de substances.

8. Procédé selon la revendication 1 pour la préparation de collections de substances de formule générale VI 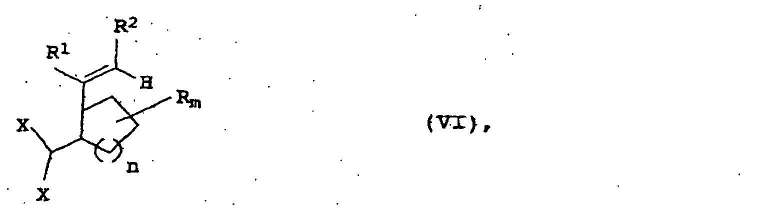 dans laquelle
X représente -COOR⁴, -CONR⁵R^{6,} -CH₂OH et
R⁴, R⁵ et R⁶ représentent chacun, indépendamment les uns des autres, l'hydrogène, un groupe alkyle en C1-C10 éventuellement substitué par un radical aromatique ou hétéroaromatique lui-même éventuellement substitué, un groupe cycloalkyle en C3-C8, un groupe aryle éventuellement substitué,
R¹, R², Rₘ, n, m ont es significations indiquées dans la revendication 1,
**caractérisé en ce que** l'on sépare les composés de formule I de la phase solide par réduction, transestérification, amidation ou catalyse basique.

9. Utilisation des composés de formule IV selon la revendication 6, pour la préparation de collections de substances.

10. Utilisation des composés de formule II selon la revendication 5 en tant que composant à CH acide dans des réactions de liaison C-C.

11. Utilisation des composés V en tant que phase solide selon la revendication 4, en tant que support fonctionnalisé pour des synthèses organiques, pour des opérations chromatographiques, pour l'immobilisation de substances actives ou d'antigènes.
